# EUROPEAN PATENT APPLICATION

(11) **EP 4 310 080 A1**
(43) Date of publication of application: **24.01.2024**
(21) Application number: 22770604.1
(22) Date of filing: 17.03.2022
(51) Int. Cl.: C07D 401/12, A61K 31/4439, A61P 35/00, A61P 19/06, A61P 17/00, A61P 3/00, A61P 9/10, A61P 29/00, A61P 1/00, A61P 11/06, A61P 37/08, A61P 19/02, A61P 37/06

(54) **POLYMORPHIC FORMS OF COMPOUND AND PREPARATION METHOD THEREFOR AND APPLICATION THEREOF**

(30) Priority: 19.03.2021 CN 202110297078
(71) Applicant: Shanghai Meiyue Biotech Development Co., Ltd., Shanghai 200120 (CN)
(72) Inventor: YE, Guozhong, Shanghai 200120 (CN); TIAN, Yong, Shanghai 200120 (CN); SUN, Zongguo, Shanghai 200120 (CN); LUAN, Linbo, Shanghai 200120 (CN); CHEN, Yongkai, Shanghai 200120 (CN); WANG, Chaodong, Shanghai 200120 (CN)
(74) Representative: dompatent von Kreisler Selting Werner - Partnerschaft von Patent- und Rechtsanwälten mbB
(86) International application number: PCT/CN2022/081514
(87) International publication number: WO 2022/194252

(57) **Abstract**

Disclosed in the present invention are polymorphic forms of a compound and a preparation method therefor and an application thereof. A crystal form III of a compound A uses Cu-Kα radiation, and X-ray powder diffraction expressed at 2θ angles has characteristic peaks at 12.15±0.20°, 15.98±0.20°, 16.62±0.20°, 17.14±0.20°, 24.32±0.20°, and 26.08±0.20°. A crystal form VII of the compound A uses Cu-Kα radiation, and X-ray powder diffraction expressed at 2θ angles has characteristic peaks at 12.94±0.20°, 14.41±0.20°, 15.64±0.20°, 17.25±0.20°, 21.75±0.20°, and 24.23±0.20°. The polymorphic forms prepared by the present invention are good in stability, and can be stably stored under the conditions of high temperature and low relative humidity.

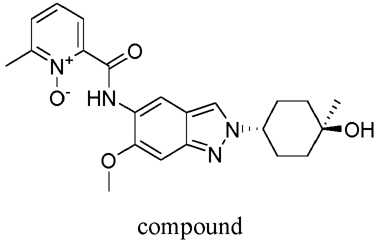

## Description

The present application claims the right of the priority of an earlier application submitted to the China National Intellectual Property Administration on March 19, 2021, with patent application number 202110297078.4 and invention title "POLYMORPH OF COMPOUND, PREPARATION METHOD THEREFOR AND USE THEREOF". The full text of the application is incorporated in this application by reference.

### TECHNICAL FIELD

The present disclosure belongs to the field of pharmaceutical crystalline forms, and relates to a polymorph of a compound, a preparation method therefor and use thereof, and particularly to a polymorph of 2-((2-(*trans*-4-hydroxy-*cis*-4-methylcyclohexyl)-6-methoxy-2*H*-indazol-5-yl)carbamoyl)-6-m ethylpyridine 1-oxide, a preparation method for the polymorph and use thereof.

### BACKGROUND

Interleukin-1 receptor-associated kinase (IRAK) is a family of serine/threonine protein kinases present in cells, with four members: IRAK1, IRAK2, IRAK-M and IRAK4. A common feature of these four members is the typical N-terminal death domain that mediates the interaction between the MyD88 family adapter protein and the central kinase domain, wherein IRAK1 and IRAK4 have kinase activity. IRAK4 is a key factor downstream of the Toll-like receptor (TLR)/interleukin-1 receptor (IL-1R)-mediated inflammatory signaling pathway. When the binding of ligand to a pathogen-specific molecule (e.g., lipopolysaccharide, polypeptide and viral DNA) is recognized by the extracellular portion of TLR, the intracellular portion recruits MyD88 and other factors to form complexes and initiate IRAK1 autophosphorylation, thereby activating downstream serine/threonine kinase TAK1, activating NF-κB and MAPK signaling pathways, producing proinflammatory cytokines, chemokines and destructive enzymes, and ultimately leading to inflammatory responses that mediate innate immunity. IL-1R is involved in host defense and hematopoiesis and serves as a bridge connecting the innate immunity and acquired immunity. (Flannery, et al., Biochem. Pharmacol., 2010, 80 (12):1981-1991).

Studies show that the excessive activation of IRAK4-dependent TLR/IL-1R signaling pathway is closely related to the onset and progression of rheumatoid arthritis. It has also been confirmed in various studies that IRAK4 activation is closely related to the onset and progression of diseases such as tumors, gout, systemic lupus erythematosus, multiple sclerosis, metabolic syndrome, atherosclerosis, myocardial infarction, sepsis, inflammatory bowel disease, asthma, and allergy (Chaudhary D, et al., J. Med. Chem. 2015, 58 (1):96-110).

At present, the patent application PCT/CN2020/117093 (priority to CN201910906833.7) that has been filed by the applicant describes a new compound that can be effectively used for preparing a medicament for treating the above IRAK-mediated and/or interleukin-1 receptor-associated disease, and especially used as a medicament for treating and/or preventing the above IRAK-mediated and/or interleukin-1 receptor-associated disease. How to develop pharmaceutical crystalline forms of such compounds suitable for drug preparation, especially crystalline forms that have improved stability, hygroscopicity and/or efficacy, and thus achieve good effects in preparing and using medicaments, has become a technical problem to be solved urgently.

### SUMMARY

In order to improve the above problem in the prior art, the present disclosure provides a polymorph of 2-((2-(*trans*-4-hydroxy-*cis*-4-methylcyclohexyl)-6-methoxy-2*H*-indazol-5-yl)carbamoyl)-6-m ethylpyridine 1-oxide shown as compound A of the following formula:

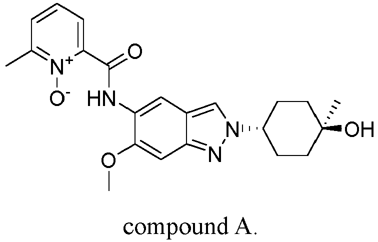

The present disclosure provides a crystalline form I of compound A, wherein the crystalline form I is characterized by X-ray powder diffraction peaks measured using Cu-Kα radiation at 2θ angles of 11.85±0.20°, 15.86±0.20°, 16.57±0.20°, 17.68±0.20°, 20.99±0.20° and 23.99±0.20°.

According to an embodiment of the present disclosure, the crystalline form I is an anhydrate of compound A.

Preferably, the crystalline form I is characterized by X-ray powder diffraction peaks measured using Cu-Kα radiation at 2θ angles of 6.02±0.20°, 11.85±0.20°, 15.86±0.20°, 16.26±0.20°, 16.57±0.20°, 17.68±0.20°, 20.99±0.20° and 23.99±0.20°.

Preferably, the crystalline form I is characterized by X-ray powder diffraction peaks measured using Cu-Kα radiation at 2θ angles of 6.02±0.20°, 11.85±0.20°, 15.86±0.20°, 16.26±0.20°, 16.57±0.20°, 17.40±0.20°, 17.68±0.20°, 18.33±0.20°, 20.99±0.20°, 23.99±0.20° and 27.76±0.20°. Preferably, the crystalline form I is characterized by X-ray powder diffraction peaks measured using Cu-Kα radiation at 2θ angles with an error range of ±0.20° as shown in Table 1:

**Table 1. XRPD analysis data for crystalline form I**

| Peak number | 2θ [ ° ] | Relative intensity (%) | Peak number | 2θ [ ° ] | Relative intensity (%) |
|---|---|---|---|---|---|
| 1 | 4.541 | 0.5 | 25 | 24.929 | 5.2 |
| 2 | 6.02 | 11.9 | 26 | 25.175 | 4.3 |
| 3 | 6.925 | 2.2 | 27 | 25.597 | 1.8 |
| 4 | 7.557 | 0.5 | 28 | 26.214 | 4.7 |
| 5 | 10.471 | 2.9 | 29 | 27.029 | 0.9 |
| 6 | 10.916 | 4.4 | 30 | 27.765 | 9.6 |
| 7 | 11.851 | 32.6 | 31 | 28.528 | 0.4 |
| 8 | 12.126 | 100 | 32 | 29.103 | 1 |
| 9 | 13.674 | 3 | 33 | 29.602 | 4.2 |
| 10 | 14.963 | 2.2 | 34 | 30.259 | 0.6 |
| 11 | 15.237 | 6.2 | 35 | 30.757 | 1.3 |
| 12 | 15.868 | 35.1 | 36 | 30.98 | 1.3 |
| 13 | 16.261 | 13.7 | 37 | 31.78 | 1 |
| 14 | 16.577 | 36.9 | 38 | 32.49 | 3.3 |
| 15 | 17.404 | 8 | 39 | 32.977 | 1.9 |
| 16 | 17.68 | 17.2 | 40 | 33.354 | 0.9 |
| 17 | 18.337 | 9.9 | 41 | 34.172 | 0.6 |
| 18 | 19.15 | 3.8 | 42 | 34.67 | 1 |
| 19 | 20.989 | 13.2 | 43 | 35.182 | 1.6 |
| 20 | 21.593 | 4.1 | 44 | 35.563 | 0.2 |
| 21 | 22.446 | 1.3 | 45 | 37.046 | 1 |
| 22 | 22.681 | 2.2 | 46 | 37.874 | 1.4 |
| 23 | 23.457 | 17.3 | 47 | 38.752 | 0.8 |
| 24 | 23.996 | 38.4 | | | |

.

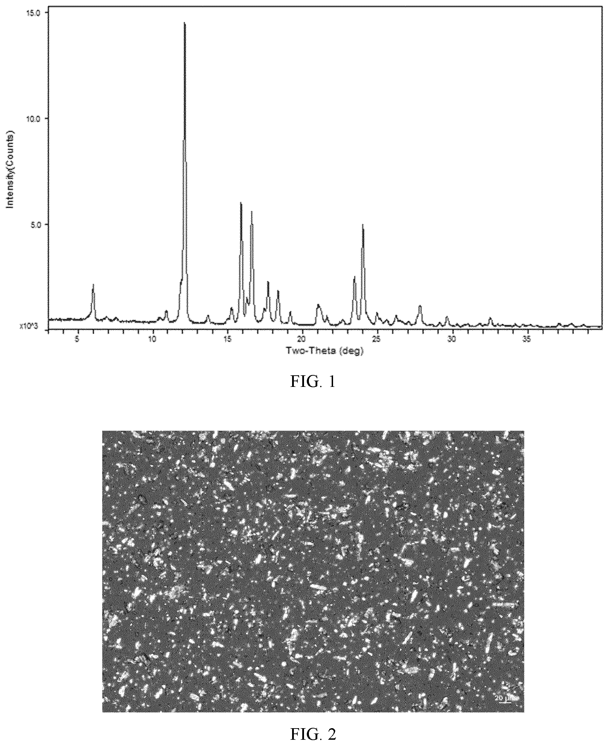

Preferably, the crystalline form I has an X-ray powder diffraction pattern substantially as shown in FIG. 1.

According to an embodiment of the present disclosure, differential scanning calorimetry (DSC) analysis of the crystalline form I shows a first endothermic peak at a peak temperature raised to near 190.70 °C.

According to an embodiment of the present disclosure, thermogravimetric analysis (TGA) of the crystalline form I shows a weight loss of about 1.2% in an interval from 140 to 200 °C.

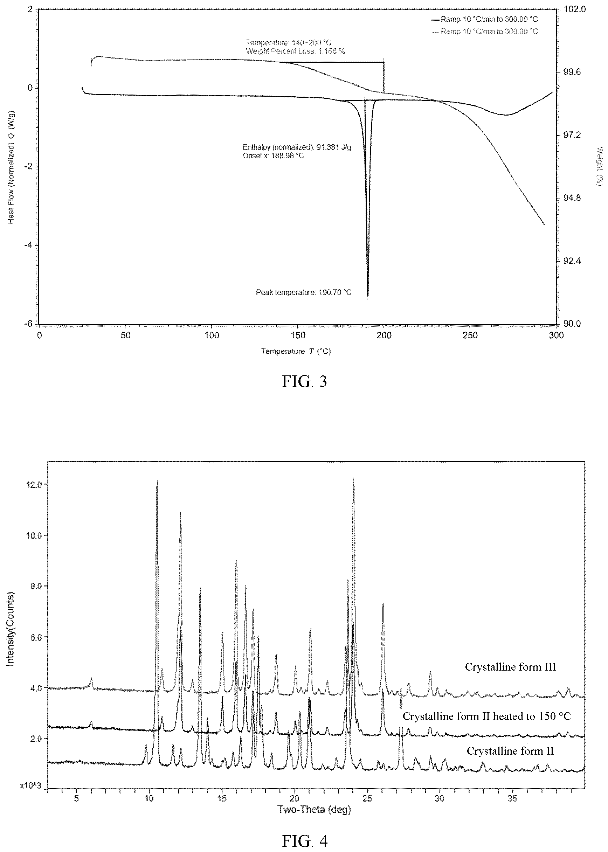

Preferably, the crystalline form I has a DSC-TGA pattern substantially as shown in FIG. 3.

According to an embodiment of the present disclosure, the crystalline form I is a crystal with irregular morphology. Preferably, the crystalline form I has a particle size of 20 µm or less. Preferably, the crystalline form I has a PLM image substantially as shown in FIG. 2.

According to an embodiment of the present disclosure, the crystalline form I has a purity of 95% or more, preferably 99% or more.

The present disclosure further provides a crystalline form II of compound A, wherein the crystalline form II is characterized by X-ray powder diffraction peaks measured using Cu-Kα radiation at 2θ angles of 13.49±0.20°, 17.51±0.20°, 17.72±0.20°, 20.97±0.20°, 23.67±0.20° and 27.32±0.20°.

According to an embodiment of the present disclosure, the crystalline form II is a toluene solvate of compound A.

Preferably, the crystalline form II is characterized by X-ray powder diffraction peaks measured using Cu-Kα radiation at 2θ angles of 13.49±0.20°, 14.03±0.20°, 17.16±0.20°, 17.51±0.20°, 17.72±0.20°, 20.97±0.20°, 23.67±0.20° and 27.32±0.20°.

Preferably, the crystalline form II is characterized by X-ray powder diffraction peaks measured using Cu-Kα radiation at 2θ angles of 13.49±0.20°, 14.03±0.20°, 17.16±0.20°, 17.51±0.20°, 17.72±0.20°, 19.58±0.20°, 19.76±0.20°, 20.36±0.20°, 20.97±0.20°, 23.67±0.20° and 27.32±0.20°.

Preferably, the crystalline form II is characterized by X-ray powder diffraction peaks measured using Cu-Kα radiation at 2θ angles with an error range of ±0.20° as shown in Table 2:

**Table 2. XRPD analysis data for crystalline form II**

| Peak number | 2θ [ ° ] | Relative intensity (%) | Peak number | 2θ [ ° ] | Relative intensity (%) |
|---|---|---|---|---|---|
| 1 | 5.205 | 1.8 | 28 | 25.743 | 3.5 |
| 2 | 9.799 | 6 | 29 | 26.097 | 2 |
| 3 | 10.537 | 100 | 30 | 26.463 | 1 |
| 4 | 11.651 | 8.1 | 31 | 26.782 | 0.7 |
| 5 | 12.178 | 5.7 | 32 | 27.317 | 30.9 |
| 6 | 13.492 | 60.8 | 33 | 27.845 | 0.3 |
| 7 | 14.028 | 19 | 34 | 28.315 | 7.4 |
| 8 | 14.317 | 0.7 | 35 | 28.563 | 5.8 |
| 9 | 15.055 | 3.5 | 36 | 29.353 | 5.7 |
| 10 | 15.211 | 4.6 | 37 | 29.654 | 2.6 |
| 11 | 15.776 | 5.3 | 38 | 30.351 | 6.3 |
| 12 | 16.287 | 9.8 | 39 | 30.707 | 1.5 |
| 13 | 17.156 | 19.1 | 40 | 31.022 | 2 |
| 14 | 17.509 | 52.1 | 41 | 31.374 | 3.3 |
| 15 | 17.719 | 53 | 42 | 31.545 | 3.5 |
| 16 | 18.427 | 3.8 | 43 | 32.95 | 4.8 |
| 17 | 19.584 | 15.5 | 44 | 33.488 | 1.3 |
| 18 | 19.767 | 18.3 | 45 | 34.198 | 0.8 |
| 19 | 20.358 | 17.6 | 46 | 34.579 | 2.3 |
| 20 | 20.976 | 27.9 | 47 | 35.222 | 0.8 |
| 21 | 21.933 | 2.1 | 48 | 35.63 | 2.3 |
| 22 | 22.278 | 1 | 49 | 36.469 | 1.6 |
| 23 | 22.853 | 3.6 | 50 | 36.705 | 2.8 |
| 24 | 23.065 | 1.3 | 51 | 37.376 | 2.9 |
| 25 | 23.667 | 68.6 | 52 | 37.929 | 0.8 |
| 26 | 24.533 | 3 | 53 | 38.567 | 1.1 |
| 27 | 25.354 | 0.2 | 54 | 39.015 | 0.6 |

.

Preferably, the crystalline form II has an X-ray powder diffraction pattern substantially as shown in FIG. 4.

According to an embodiment of the present disclosure, differential scanning calorimetry (DSC) analysis of the crystalline form II shows a first endothermic peak at a peak temperature raised to near 136.92 °C and a second endothermic peak at a peak temperature raised to near 189.27 °C.

According to an embodiment of the present disclosure, thermogravimetric analysis (TGA) of the crystalline form II shows a weight loss of about 9.6% from 100 to 160 °C.

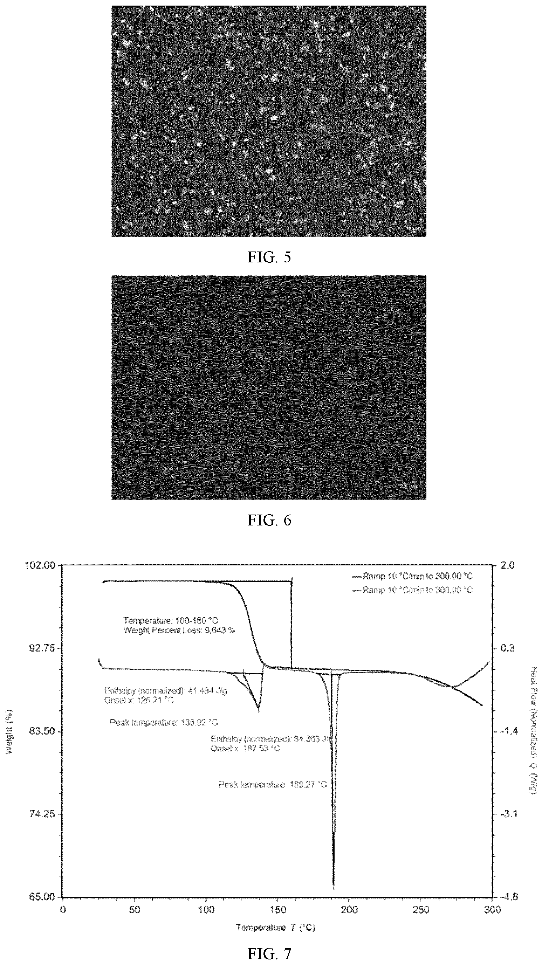

Preferably, the crystalline form II has a DSC-TGA pattern substantially as shown in FIG. 7.

According to an embodiment of the present disclosure, the crystalline form II is a crystal with irregular morphology. Preferably, the crystalline form II has a particle size of less than 10 µm. Preferably, the crystalline form II has a PLM image substantially as shown in FIG. 5.

According to an embodiment of the present disclosure, the crystalline form II has a purity of 95% or more, preferably 99% or more.

The present disclosure further provides a crystalline form III of compound A, wherein the crystalline form III is characterized by X-ray powder diffraction peaks measured using Cu-Kα radiation at 2θ angles of 12.15±0.20°, 15.98±0.20°, 16.62±0.20°, 17.14±0.20°, 24.32±0.20° and 26.08±0.20°.

According to an embodiment of the present disclosure, the crystalline form III is an anhydrate of compound A.

Preferably, the crystalline form III is characterized by X-ray powder diffraction peaks measured using Cu-Kα radiation at 2θ angles of 12.15±0.20°, 15.04±0.20°, 15.98±0.20°, 16.62±0.20°, 17.14±0.20°, 21.09±0.20°, 24.32±0.20° and 26.08±0.20°.

Preferably, the crystalline form III is characterized by X-ray powder diffraction peaks measured using Cu-Kα radiation at 2θ angles of 12.15±0.20°, 15.04±0.20°, 15.98±0.20°, 16.62±0.20°, 17.14±0.20°, 18.74±0.20°, 21.09±0.20°, 23.51±0.20°, 24.32±0.20° and 26.08±0.20°.

Preferably, the crystalline form III is characterized by X-ray powder diffraction peaks measured using Cu-Kα radiation at 2θ angles with an error range of ±0.20° as shown in Table 3:

**Table 3. XRPD analysis data for crystalline form III**

| Peak number | 2θ [ ° ] | Relative intensity (%) | Peak number | 2θ [ ° ] | Relative intensity (%) |
|---|---|---|---|---|---|
| 1 | 6.021 | 5.2 | 22 | 26.083 | 37.3 |
| 2 | 10.879 | 8.2 | 23 | 26.688 | 1.3 |
| 3 | 12.152 | 77.4 | 24 | 27.106 | 1 |
| 4 | 12.978 | 3.6 | 25 | 27.83 | 5.8 |
| 5 | 15.04 | 20.5 | 26 | 28.262 | 0.2 |
| 6 | 15.986 | 47.8 | 27 | 29.314 | 9.1 |
| 7 | 16.617 | 34.2 | 28 | 29.824 | 3.2 |
| 8 | 17.141 | 27 | 29 | 30.429 | 3.6 |
| 9 | 18.323 | 1.5 | 30 | 30.818 | 1.5 |
| 10 | 18.742 | 13.7 | 31 | 31.926 | 4.9 |
| 11 | 20.068 | 8.7 | 32 | 32.373 | 3.1 |
| 12 | 20.449 | 3.5 | 33 | 32.583 | 2.3 |
| 13 | 20.765 | 3.2 | 34 | 33.186 | 1.4 |
| 14 | 21.092 | 21.8 | 35 | 33.777 | 2.5 |
| 15 | 21.645 | 2.2 | 36 | 34.392 | 0.6 |
| 16 | 22.262 | 5.1 | 37 | 35.418 | 2.4 |
| 17 | 22.97 | 0.4 | 38 | 36.023 | 1.7 |
| 18 | 23.51 | 19.6 | 39 | 36.6 | 2 |
| 19 | 24.048 | 100 | 40 | 38.138 | 4 |
| 20 | 24.323 | 50.8 | 41 | 38.806 | 4.3 |
| 21 | 24.598 | 8.3 | 42 | 39.306 | 2 |

.

Preferably, the crystalline form III has an X-ray powder diffraction pattern substantially as shown in FIG. 4.

According to an embodiment of the present disclosure, differential scanning calorimetry (DSC) analysis of the crystalline form III shows a first endothermic peak at a peak temperature raised to near 188.81 °C.

According to an embodiment of the present disclosure, thermogravimetric analysis (TGA) of the crystalline form III shows almost no weight loss below 180 °C.

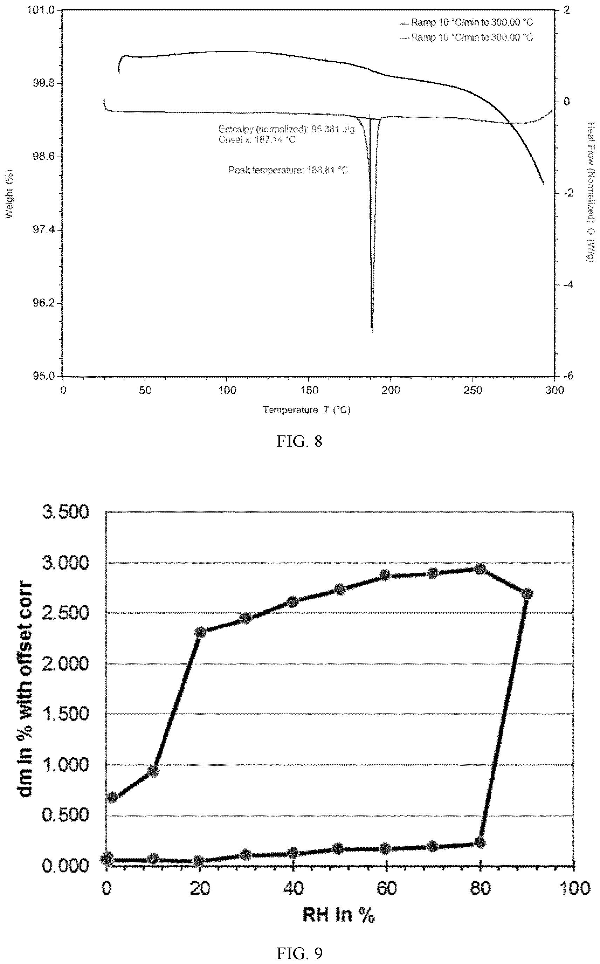

Preferably, the crystalline form III has a DSC-TGA pattern substantially as shown in FIG. 8.

According to an embodiment of the present disclosure, the crystalline form III is a crystal with irregular morphology. Preferably, the crystalline form III has a particle size of less than 5 µm. Preferably, the crystalline form III has a PLM image substantially as shown in FIG. 6.

According to an embodiment of the present disclosure, the crystalline form III has a purity of 95% or more, preferably 99% or more.

The present disclosure further provides a crystalline form IV of compound A, wherein the crystalline form IV is characterized by X-ray powder diffraction peaks measured using Cu-Kα radiation at 2θ angles of 5.38±0.20°, 6.68±0.20°, 9.76±0.20°, 19.69±0.20°, 27.48±0.20° and 29.65±0.20°.

According to an embodiment of the present disclosure, the crystalline form IV is a hydrate of compound A. Preferably, the crystalline form IV is a monohydrate of compound A. Preferably, the crystalline form IV has a water content of 4.2wt%.

Preferably, the crystalline form IV is characterized by X-ray powder diffraction peaks measured using Cu-Kα radiation at 2θ angles of 5.38±0.20°, 6.68±0.20°, 9.76±0.20°, 19.69±0.20°, 20.13±0.20°, 25.53±0.20°, 27.48±0.20°, 27.81±0.20° and 29.65±0.20°.

Preferably, the crystalline form IV is characterized by X-ray powder diffraction peaks measured using Cu-Kα radiation at 2θ angles with an error range of ±0.20° as shown in Table 4:

**Table 4. XRPD analysis data for crystalline form IV**

| Peak number | 2θ [ ° ] | Relative intensity (%) | Peak number | 2θ [ ° ] | Relative intensity (%) |
|---|---|---|---|---|---|
| 1 | 4.584 | 1.3 | 24 | 23.916 | 4.5 |
| 2 | 5.377 | 69.2 | 25 | 24.677 | 2.8 |
| 3 | 6.677 | 28.9 | 26 | 25.529 | 10.8 |
| 4 | 9.762 | 27.6 | 27 | 25.952 | 8.1 |
| 5 | 10.024 | 52 | 28 | 26.426 | 0.7 |
| 6 | 10.736 | 7.8 | 29 | 26.959 | 3 |
| 7 | 12.442 | 2.7 | 30 | 27.163 | 4.4 |
| 8 | 13.425 | 3.2 | 31 | 27.476 | 11.6 |
| 9 | 13.753 | 6.4 | 32 | 27.815 | 10.6 |
| 10 | 14.818 | 100 | 33 | 28.633 | 2 |
| 11 | 15.355 | 5.1 | 34 | 29.655 | 12.4 |
| 12 | 16.183 | 7.9 | 35 | 31.032 | 5.6 |
| 13 | 16.604 | 9.3 | 36 | 31.776 | 1.2 |
| 14 | 16.881 | 2.9 | 37 | 32.752 | 1.1 |
| 15 | 17.494 | 2.9 | 38 | 33.988 | 0.7 |
| 16 | 18.638 | 7.7 | 39 | 35.007 | 2.8 |
| 17 | 19.689 | 63.4 | 40 | 35.351 | 2 |
| 18 | 20.134 | 11 | 41 | 36.377 | 2.2 |
| 19 | 20.519 | 0.9 | 42 | 37.479 | 3 |
| 20 | 21.414 | 3 | 43 | 38.361 | 2.2 |
| 21 | 21.672 | 2.9 | 44 | 38.465 | 2.2 |
| 22 | 22.957 | 7.2 | 45 | 39.566 | 2.1 |
| 23 | 23.372 | 3.7 | | | |

.

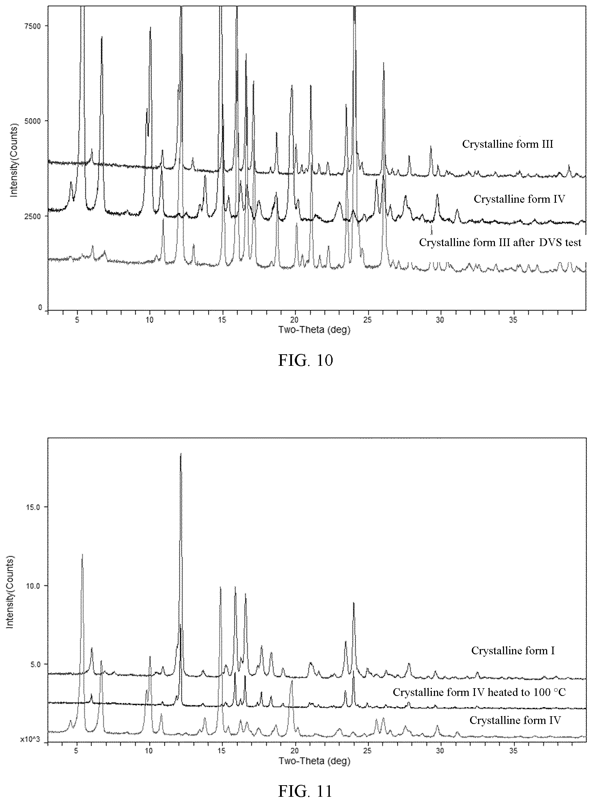

Preferably, the crystalline form IV has an X-ray powder diffraction pattern substantially as shown in FIG. 11.

According to an embodiment of the present disclosure, differential scanning calorimetry (DSC) analysis of the crystalline form IV shows a first endothermic peak at a peak temperature raised to near 77.01 °C, a second endothermic peak at a peak temperature raised to near 190.76 °C, a third endothermic peak at a peak temperature raised to near 201.77 °C, a fourth endothermic peak at a peak temperature raised to near 215.93 °C and a fifth endothermic peak at a peak temperature raised to near 218.05 °C. The crystalline form IV may undergo polymorphic transition during heating.

According to an embodiment of the present disclosure, thermogravimetric analysis (TGA) of the crystalline form IV shows a weight loss of about 16.9% from room temperature to 150 °C.

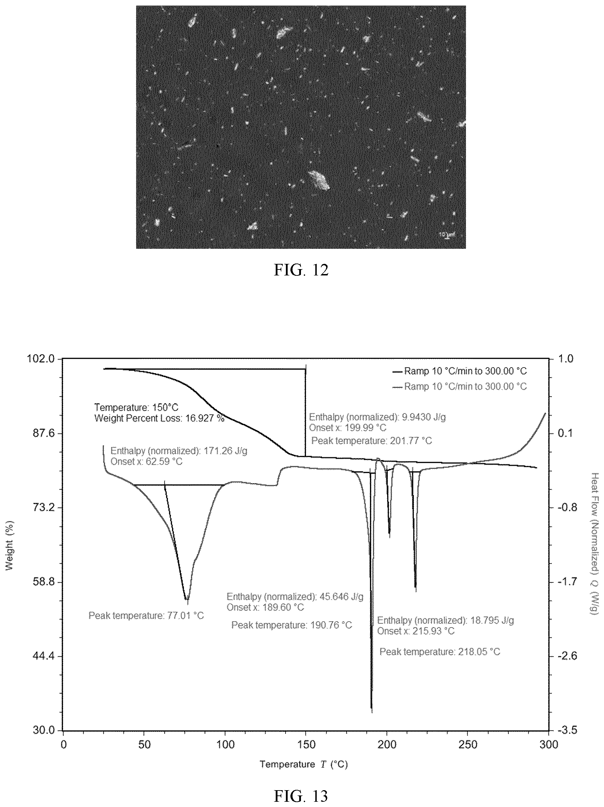

Preferably, the crystalline form IV has a DSC-TGA pattern substantially as shown in FIG. 13.

According to an embodiment of the present disclosure, the crystalline form IV is a crystal with irregular morphology. Preferably, the crystalline form IV has a particle size of less than 10 µm. Preferably, the crystalline form IV has a PLM image substantially as shown in FIG. 12.

According to an embodiment of the present disclosure, the crystalline form IV has a purity of 95% or more, preferably 99% or more.

The present disclosure further provides a crystalline form V of compound A, wherein the crystalline form V is characterized by X-ray powder diffraction peaks measured using Cu-Kα radiation at 2θ angles of 7.11±0.20°, 9.62±0.20°, 14.07±0.20°, 19.23±0.20°, 21.59±0.20° and 25.65±0.20°.

According to an embodiment of the present disclosure, the crystalline form V is an acetonitrile solvate of compound A.

Preferably, the crystalline form V is characterized by X-ray powder diffraction peaks measured using Cu-Kα radiation at 2θ angles of 7.11±0.20°, 9.62±0.20°, 11.23±0.20°, 14.07±0.20°, 19.23±0.20°, 21.59±0.20°, 22.98±0.20° and 25.65±0.20°.

Preferably, the crystalline form V is characterized by X-ray powder diffraction peaks measured using Cu-Kα radiation at 2θ angles of 7.11±0.20°, 9.62±0.20°, 11.23±0.20°, 14.07±0.20°, 19.23±0.20°, 21.59±0.20°, 22.05±0.20°, 22.98±0.20° and 25.65±0.20°.

Preferably, the crystalline form V is characterized by X-ray powder diffraction peaks measured using Cu-Kα radiation at 2θ angles with an error range of ±0.20° as shown in Table 5:

**Table 5. XRPD analysis data for crystalline form V**

| Peak number | 2θ [ ° ] | Relative intensity (%) | Peak number | 2θ [ ° ] | Relative intensity (%) |
|---|---|---|---|---|---|
| 1 | 5.547 | 0.6 | 15 | 25.649 | 18.5 |
| 2 | 7.11 | 98 | 16 | 26.371 | 1.5 |
| 3 | 8.514 | 4.2 | 17 | 27.582 | 1.6 |
| 4 | 9.617 | 26.1 | 18 | 28.447 | 2.3 |
| 5 | 11.234 | 16.2 | 19 | 29.246 | 1.5 |
| 6 | 12.556 | 0.3 | 20 | 30.219 | 2.6 |
| 7 | 14.069 | 43.3 | 21 | 32.477 | 4.1 |
| 8 | 16.038 | 100 | 22 | 33.462 | 0.9 |
| 9 | 17.587 | 1.1 | 23 | 34.307 | 0.6 |
| 10 | 19.229 | 16.9 | 24 | 36.429 | 3.7 |
| 11 | 21.592 | 26 | 25 | 37.624 | 1.2 |
| 12 | 22.051 | 5.8 | 26 | 38.187 | 0.5 |
| 13 | 22.985 | 8.1 | 27 | 39.331 | 3.7 |
| 14 | 23.602 | 2.1 | 28 | 39.632 | 3.4 |

.

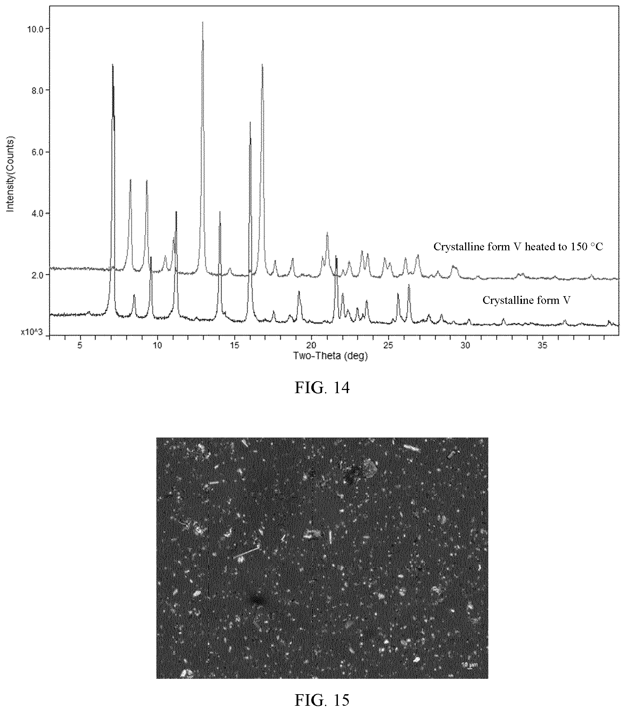

Preferably, the crystalline form V has an X-ray powder diffraction pattern substantially as shown in FIG. 14.

According to an embodiment of the present disclosure, differential scanning calorimetry (DSC) analysis of the crystalline form V shows a first endothermic peak at a peak temperature raised to near 135.05 °C, a second endothermic peak at a peak temperature raised to near 192.24 °C and a third endothermic peak at a peak temperature raised to near 218.33 °C. The crystalline form V may undergo polymorphic transition during heating.

According to an embodiment of the present disclosure, thermogravimetric analysis (TGA) of the crystalline form V shows a weight loss of about 6.4% from 70 to 150 °C.

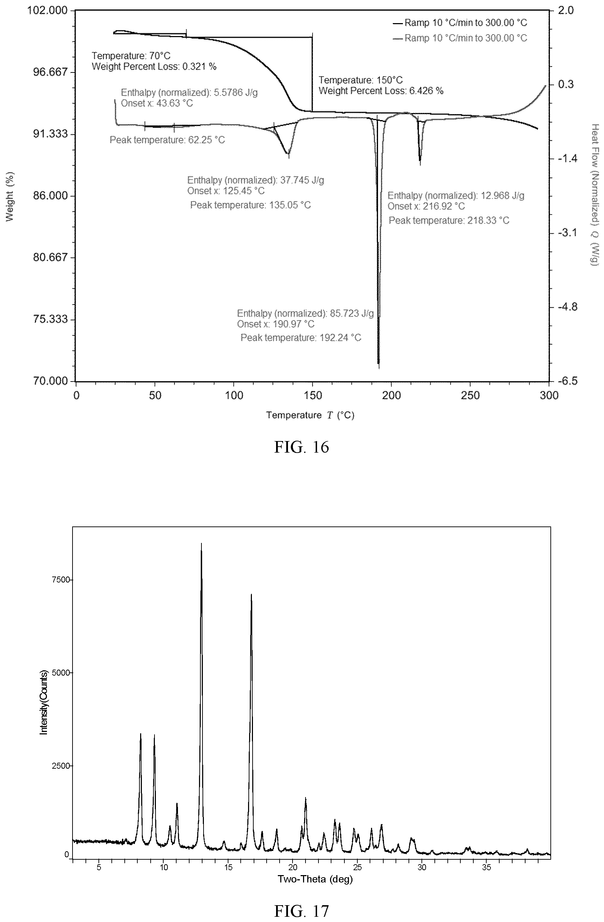

Preferably, the crystalline form V has a DSC-TGA pattern substantially as shown in FIG. 16.

According to an embodiment of the present disclosure, the crystalline form V is a crystal with irregular morphology. Preferably, the crystalline form V has a particle size of less than 10 µm. Preferably, the crystalline form V has a PLM image substantially as shown in FIG. 15.

According to an embodiment of the present disclosure, the crystalline form V has a purity of 95% or more, preferably 99% or more.

The present disclosure further provides a crystalline form IX of compound A, wherein the crystalline form IX is characterized by X-ray powder diffraction peaks measured using Cu-Kα radiation at 2θ angles of 8.26±0.20°, 9.33±0.20°, 11.07±0.20°, 16.81±0.20°, 20.73±0.20° and 21.01±0.20°.

Preferably, the crystalline form IX is characterized by X-ray powder diffraction peaks measured using Cu-Kα radiation at 2θ angles of 8.26±0.20°, 9.33±0.20°, 11.07±0.20°, 16.81±0.20°, 20.73±0.20°, 21.01±0.20°, 23.27±0.20° and 26.87±0.20°.

Preferably, the crystalline form IX is characterized by X-ray powder diffraction peaks measured using Cu-Kα radiation at 2θ angles of 8.26±0.20°, 9.33±0.20°, 11.07±0.20°, 16.81±0.20°, 20.73±0.20°, 21.01±0.20°, 23.27±0.20°, 24.76±0.20°, 25.09±0.20°, 26.87±0.20°, 29.17±0.20° and 29.42±0.20°.

Preferably, the crystalline form IX is characterized by X-ray powder diffraction peaks measured using Cu-Kα radiation at 2θ angles with an error range of ±0.20° as shown in Table 6:

**Table 6. XRPD analysis data for crystalline form IX**

| Peak number | 2θ [ ° ] | Relative intensity (%) | Peak number | 2θ [ ° ] | Relative intensity (%) |
|---|---|---|---|---|---|
| 1 | 5.482 | 1 | 19 | 23.641 | 11.6 |
| 2 | 8.265 | 43.8 | 20 | 24.757 | 12.5 |
| 3 | 9.329 | 35.9 | 21 | 25.086 | 12.2 |
| 4 | 10.524 | 9.6 | 22 | 25.769 | 0.2 |
| 5 | 11.075 | 14.4 | 23 | 26.097 | 7.5 |
| 6 | 12.953 | 100 | 24 | 26.871 | 12.8 |
| 7 | 14.686 | 3.5 | 25 | 27.419 | 0.3 |
| 8 | 16.814 | 95.7 | 26 | 27.75 | 1 |
| 9 | 17.666 | 5.7 | 27 | 28.183 | 3.2 |
| 10 | 18.296 | 1 | 28 | 29.169 | 10.6 |
| 11 | 18.795 | 8.7 | 29 | 29.417 | 10.7 |
| 12 | 19.423 | 1.2 | 30 | 30.784 | 1.1 |
| 13 | 19.834 | 1 | 31 | 31.532 | 0.3 |
| 14 | 20.726 | 16.7 | 32 | 33.448 | 2.5 |
| 15 | 21.015 | 24.7 | 33 | 33.711 | 4.5 |
| 16 | 22.051 | 2 | 34 | 35.81 | 1.3 |
| 17 | 22.471 | 7.5 | 35 | 37.164 | 1 |
| 18 | 23.274 | 13.5 | 36 | 38.175 | 1.4 |

.

Preferably, the crystalline form IX has an X-ray powder diffraction pattern substantially as shown in FIG. 17.

According to an embodiment of the present disclosure, the crystalline form IX is an anhydrate of compound A.

According to an embodiment of the present disclosure, differential scanning calorimetry (DSC) analysis of the crystalline form IX shows a first endothermic peak at a peak temperature raised to near 192.04 °C, a second endothermic peak at a peak temperature raised to near 201.20 °C and a third endothermic peak at a peak temperature raised to near 217.55 °C. The crystalline form IX may undergo polymorphic transition during heating.

According to an embodiment of the present disclosure, thermogravimetric analysis (TGA) of the crystalline form IX shows almost no weight loss below 180 °C.

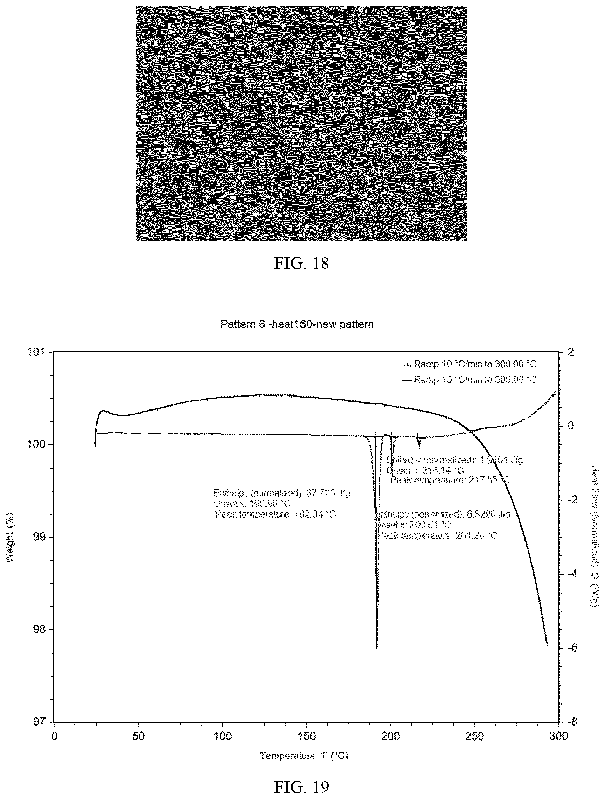

Preferably, the crystalline form IX has a DSC-TGA pattern substantially as shown in FIG. 19.

According to an embodiment of the present disclosure, the crystalline form IX is a crystal with irregular morphology. Preferably, the crystalline form IX has a particle size of less than 5 µm. Preferably, the crystalline form IX has a PLM image substantially as shown in FIG. 18.

According to an embodiment of the present disclosure, the crystalline form IX has a purity of 95% or more, preferably 99% or more.

The present disclosure further provides a crystalline form VI of compound A, wherein the crystalline form VI is characterized by X-ray powder diffraction peaks measured using Cu-Kα radiation at 2θ angles of 5.23±0.20°, 5.63±0.20°, 6.90±0.20°, 13.77±0.20°, 18.14±0.20° and 25.85±0.20°. Preferably, the crystalline form VI is characterized by X-ray powder diffraction peaks measured using Cu-Kα radiation at 2θ angles of 5.23±0.20°, 5.63±0.20°, 6.90±0.20°, 13.77±0.20°, 16.26±0.20°, 18.14±0.20°, 18.37±0.20° and 25.85±0.20°.

Preferably, the crystalline form VI is characterized by X-ray powder diffraction peaks measured using Cu-Kα radiation at 2θ angles of 5.23±0.20°, 5.63±0.20°, 6.90±0.20°, 8.08±0.20°, 13.77±0.20°, 15.78±0.20°, 16.26±0.20°, 18.14±0.20°, 18.37±0.20°, 20.87±0.20°, 25.40±0.20° and 25.85±0.20°.

Preferably, the crystalline form VI is characterized by X-ray powder diffraction peaks measured using Cu-Kα radiation at 2θ angles with an error range of ±0.20° as shown in Table 7:

**Table 7. XRPD analysis data for crystalline form VI**

| Peak number | 2θ [ ° ] | Relative intensity (%) | Peak number | 2θ [ ° ] | Relative intensity (%) |
|---|---|---|---|---|---|
| 1 | 3.475 | 0.9 | 20 | 18.375 | 20.1 |
| 2 | 4.537 | 3.4 | 21 | 18.785 | 0.8 |
| 3 | 5.232 | 39.2 | 22 | 19.493 | 0.7 |
| 4 | 5.627 | 31.6 | 23 | 20.247 | 0.5 |
| 5 | 6.898 | 44.5 | 24 | 20.871 | 14.2 |
| 6 | 8.081 | 12.9 | 25 | 21.406 | 0.8 |
| 7 | 9.013 | 7.1 | 26 | 22.186 | 1.5 |
| 8 | 10.354 | 100 | 27 | 22.458 | 2.4 |
| 9 | 11.325 | 2.2 | 28 | 22.913 | 1.3 |
| 10 | 12.43 | 2.2 | 29 | 23.233 | 2 |
| 11 | 13.164 | 4.3 | 30 | 23.826 | 0.6 |
| 12 | 13.767 | 20.6 | 31 | 24.546 | 1.3 |
| 13 | 14.926 | 0.8 | 32 | 25.401 | 10.1 |
| 14 | 15.775 | 12.5 | 33 | 25.848 | 20.9 |
| 15 | 16.262 | 19.2 | 34 | 26.306 | 9.3 |
| 16 | 16.906 | 0.3 | 35 | 28.947 | 1.1 |
| 17 | 17.273 | 3 | 36 | 29.64 | 1.2 |
| 18 | 17.746 | 2.2 | 37 | 31.611 | 1 |
| 19 | 18.139 | 20.9 | | | |

.

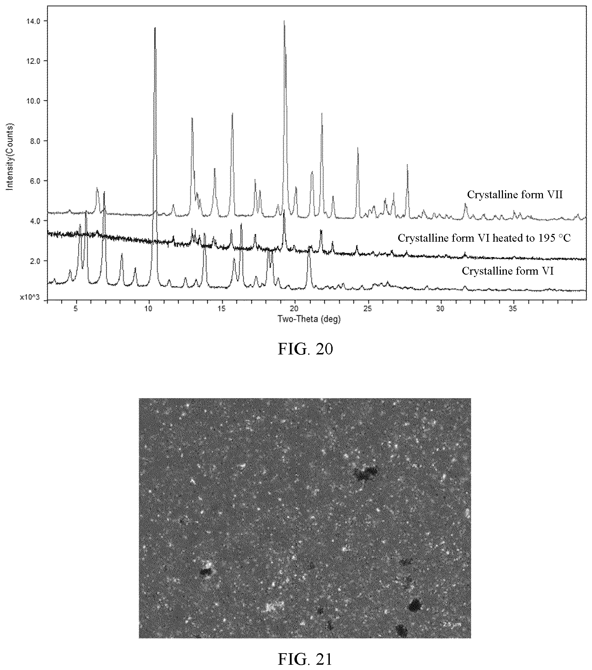

Preferably, the crystalline form VI has an X-ray powder diffraction pattern substantially as shown in FIG. 20.

According to an embodiment of the present disclosure, the crystalline form VI is a methanol solvate/hydrate of compound A.

According to an embodiment of the present disclosure, differential scanning calorimetry (DSC) analysis of the crystalline form VI shows an endothermic peak at a peak temperature raised to near 201.31 °C.

According to an embodiment of the present disclosure, thermogravimetric analysis (TGA) of the crystalline form VI shows a weight loss of about 9.5% from room temperature to 130 °C.

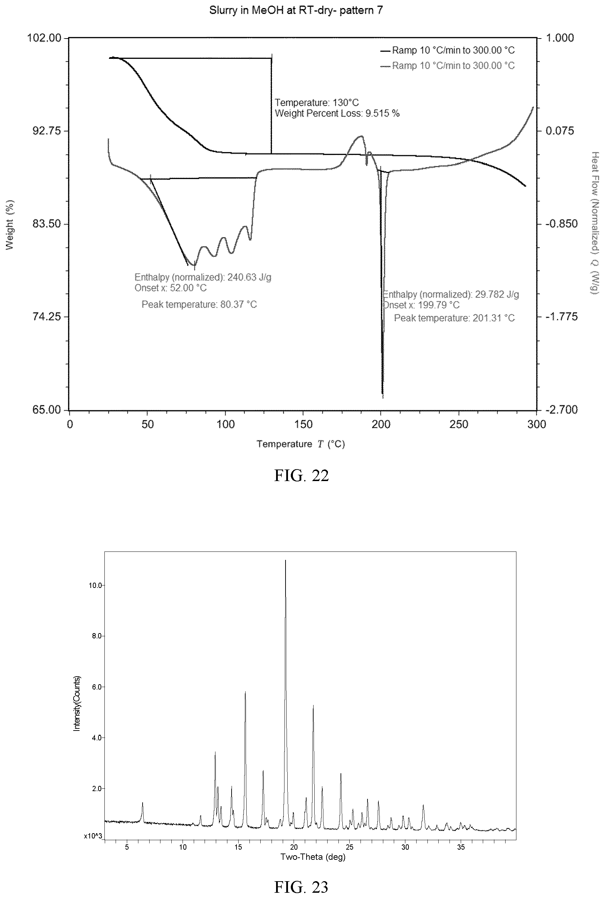

Preferably, the crystalline form VI has a DSC-TGA pattern substantially as shown in FIG. 22.

According to an embodiment of the present disclosure, the crystalline form VI is a crystal with irregular morphology. Preferably, the crystalline form VI has a particle size of less than 5 µm. Preferably, the crystalline form VI has a PLM image substantially as shown in FIG. 21.

According to an embodiment of the present disclosure, the crystalline form VI has a purity of 95% or more, preferably 99% or more.

The present disclosure further provides a crystalline form VII of compound A, wherein the crystalline form VII is characterized by X-ray powder diffraction peaks measured using Cu-Kα radiation at 2θ angles of 12.94±0.20°, 14.41±0.20°, 15.64±0.20°, 17.25±0.20°, 21.75±0.20° and 24.23±0.20°.

According to an embodiment of the present disclosure, the crystalline form VII is an anhydrate of compound A.

Preferably, the crystalline form VII is characterized by X-ray powder diffraction peaks measured using Cu-Kα radiation at 2θ angles of 12.94±0.20°, 13.18±0.20°, 14.41±0.20°, 15.64±0.20°, 17.25±0.20°, 21.75±0.20°, 22.54±0.20° and 24.23±0.20°.

Preferably, the crystalline form VII is characterized by X-ray powder diffraction peaks measured using Cu-Kα radiation at 2θ angles of 12.94±0.20°, 13.18±0.20°, 14.41±0.20°, 15.64±0.20°, 17.25±0.20°, 21.11±0.20°, 21.75±0.20°, 22.54±0.20°, 24.23±0.20°, 26.62±0.20° and 31.64±0.20°.

Preferably, the crystalline form VII is characterized by X-ray powder diffraction peaks measured using Cu-Kα radiation at 2θ angles with an error range of ±0.20° as shown in Table 8:

**Table 8. XRPD analysis data for crystalline form VII**

| Peak number | 2θ [ ° ] | Relative intensity (%) | Peak number | 2θ [ ° ] | Relative intensity (%) |
|---|---|---|---|---|---|
| 1 | 6.413 | 8 | 25 | 25.82 | 1.7 |
| 2 | 10.957 | 1.1 | 26 | 26.135 | 6.1 |
| 3 | 11.625 | 3.1 | 27 | 26.385 | 3 |
| 4 | 12.939 | 25.9 | 28 | 26.621 | 10.5 |
| 5 | 13.176 | 12.7 | 29 | 27.328 | 0.2 |
| 6 | 13.451 | 5.9 | 30 | 27.618 | 9.2 |
| 7 | 14.409 | 16.3 | 31 | 28.436 | 2 |
| 8 | 14.567 | 9.9 | 32 | 28.724 | 4.6 |
| 9 | 15.644 | 41.4 | 33 | 29.431 | 1.4 |
| 10 | 17.246 | 18.2 | 34 | 29.825 | 5.9 |
| 11 | 17.522 | 6.2 | 35 | 30.35 | 4.9 |
| 12 | 17.655 | 4.4 | 36 | 30.624 | 0.8 |
| 13 | 18.783 | 1.4 | 37 | 31.637 | 11.1 |
| 14 | 19.255 | 100 | 38 | 32.123 | 2.6 |
| 15 | 19.732 | 3.4 | 39 | 32.856 | 1.5 |
| 16 | 19.94 | 5.1 | 40 | 33.763 | 3.9 |
| 17 | 21.106 | 10.3 | 41 | 34.092 | 1.4 |
| 18 | 21.75 | 41 | 42 | 34.973 | 4.4 |
| 19 | 22.001 | 3.4 | 43 | 35.367 | 2.8 |
| 20 | 22.539 | 12.6 | 44 | 35.841 | 2.9 |
| 21 | 24.231 | 18 | 45 | 37.7 | 1.2 |
| 22 | 24.704 | 0.7 | 46 | 38.242 | 1.6 |
| 23 | 25.058 | 3.6 | 47 | 39.185 | 2.1 |
| 24 | 25.308 | 7 | 48 | 39.307 | 2.1 |

.

Preferably, the crystalline form VII has an X-ray powder diffraction pattern substantially as shown in FIG. 23.

According to an embodiment of the present disclosure, differential scanning calorimetry (DSC) analysis of the crystalline form VII has an endothermic peak at a peak temperature raised to near 201.07 °C.

According to an embodiment of the present disclosure, thermogravimetric analysis (TGA) of the crystalline form VII shows almost no weight loss below 200 °C, for example, almost no weight loss below 180 °C.

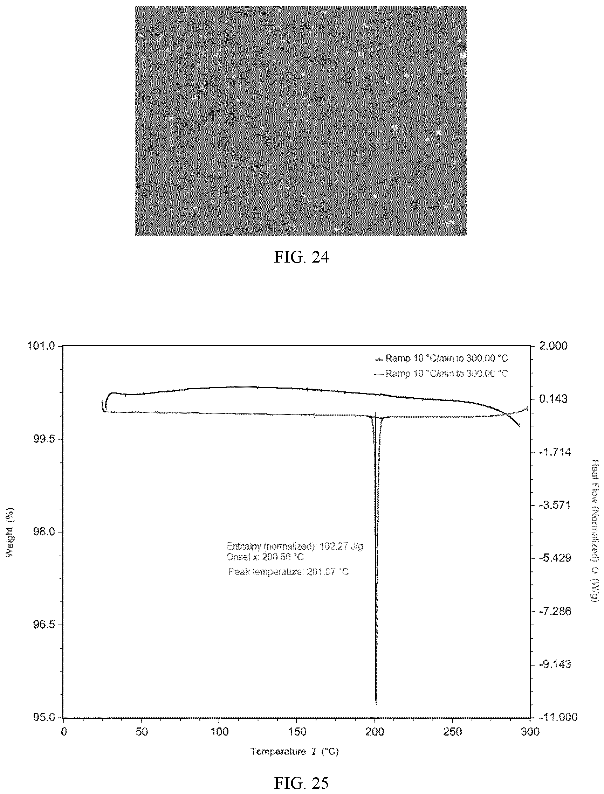

Preferably, the crystalline form VII has a DSC-TGA pattern substantially as shown in FIG. 25.

According to an embodiment of the present disclosure, the crystalline form VII is a crystal with irregular morphology. Preferably, the crystalline form VII has a particle size of less than 5 µm. Preferably, the crystalline form VII has a PLM image substantially as shown in FIG. 24.

According to an embodiment of the present disclosure, the crystalline form VII has a purity of 95% or more, preferably 99% or more.

The present disclosure further provides a preparation method for the polymorph of compound A.

According to an embodiment of the present disclosure, a preparation method for the crystalline form I comprises the following steps:

mixing compound A with a first alcohol solvent and an ether solvent, heating and stirring the mixture until complete dissolution is achieved, and performing cooling, filtration and drying to give the crystalline form I.

According to an embodiment of the present disclosure, the first alcohol solvent may be selected from ethanol and/or isopropanol, preferably ethanol.

According to an embodiment of the present disclosure, the ether solvent may be selected from methyl tert-butyl ether and/or n-heptane, preferably methyl tert-butyl ether.

According to an embodiment of the present disclosure, a mass-to-volume ratio of compound A to the first alcohol solvent to the ether solvent is 1 g : (10-20) mL : (3-8) mL, preferably 1 g : (12-18) mL : (4-6) mL, and exemplarily 1 g : 15 mL : 5 mL.

According to an embodiment of the present disclosure, the mixture is heated to a temperature of 50-70 °C, preferably 55-65 °C, and exemplarily 50 °C.

According to an embodiment of the present disclosure, the mixture is heated and stirred for a time period of 1-5 h, preferably 2-4 h, and exemplarily 3 h.

According to an embodiment of the present disclosure, the mixture is cooled to a temperature of 0-10 °C before being filtered.

According to an exemplary embodiment of the present disclosure, the preparation method for the crystalline form I comprises the following steps: adding compound A to a solvent mixture of ethanol and methyl tert-butyl ether, heating and stirring the mixture, performing filtration after cooling, and performing drying *in vacuo* to give the crystalline form I, wherein
a mass-to-volume ratio of compound A to ethanol to methyl tert-butyl ether is 1 g : (10-20) mL : (3-8) mL.

The present disclosure further provides another preparation method for the crystalline form I comprising the following step: heating the crystalline form IV to give the crystalline form I.

According to an embodiment of the present disclosure, the crystalline form IV is heated to such a temperature that the solvent therein can be completely removed. Preferably, the solvent in the crystalline form IV is water. Preferably, the crystalline form IV is heated to 100 °C.

The present disclosure further provides a preparation method for the above crystalline form II comprising the following steps:
mixing compound A with an aromatic solvent, stirring the mixture at room temperature until complete dissolution is achieved, and performing filtration to give the crystalline form II.

According to an embodiment of the present disclosure, the aromatic solvent is selected from toluene.

According to an embodiment of the present disclosure, a mass-to-volume ratio of compound A to the aromatic solvent is 1 g : (10-20) mL, preferably 1 g : (12-18) mL, and exemplarily 1 g : 15 mL.

According to an embodiment of the present disclosure, the room temperature refers to 15-30 °C, preferably 20-25 °C.

According to an embodiment of the present disclosure, the mixture is stirred at room temperature for a time period of 1-5 h, e.g., 3 h.

According to an exemplary embodiment of the present disclosure, the preparation method for the crystalline form II comprises the following steps:
mixing compound A with toluene, stirring the mixture at room temperature until complete dissolution is achieved, and performing filtration to give the crystalline form II, wherein
a mass-to-volume ratio of compound A to the aromatic solvent is 1 g : (10-20) mL.

The present disclosure further provides a preparation method for the above crystalline form III comprising the following step: heating the crystalline form II to give the crystalline form III.

According to an embodiment of the present disclosure, the crystalline form II is heated to such a temperature that the aromatic solvent therein can be completely removed. For example, the crystalline form II is heated to a temperature of 100-160 °C.

The present disclosure further provides a preparation method for the above crystalline form IV comprising the following steps:
mixing compound A with a second alcohol solvent and water, stirring the mixture at room temperature until complete dissolution is achieved, and performing filtration and drying to give the crystalline form IV

According to an embodiment of the present disclosure, the second alcohol solvent is selected from isopropanol.

According to an embodiment of the present disclosure, a mass-to-volume ratio of compound A to the second alcohol solvent to water is 1 g : (1-10) mL : (1-10) mL, preferably 1 g : (3-8) mL : (3-8) mL, and exemplarily 1 g : 5 mL : 5 mL.

According to an embodiment of the present disclosure, the room temperature refers to 15-30 °C, preferably 20-25 °C.

According to an embodiment of the present disclosure, the mixture is stirred at room temperature for a time period of 1-5 h, e.g., 3 h.

According to an exemplary embodiment of the present disclosure, the preparation method for the crystalline form IV comprises the following steps:

mixing compound A with isopropanol and water, stirring the mixture at room temperature until complete dissolution is achieved, and performing filtration and drying *in vacuo* to give the crystalline form IV, wherein
a mass-to-volume ratio of compound A to isopropanol to water is 1 g : 5 mL : 5 mL.

The present disclosure further provides a preparation method for the above crystalline form V comprising the following steps:
mixing compound A with a nitrile solvent, stirring the mixture at room temperature until complete dissolution is achieved, and performing filtration and drying to give the crystalline form V

According to an embodiment of the present disclosure, the nitrile solvent is selected from acetonitrile.

According to an embodiment of the present disclosure, a mass-to-volume ratio of compound A to the nitrile solvent is 1 g : (5-15) mL, preferably 1 g : (8-12) mL, and exemplarily 1 g : 10 mL.

According to an embodiment of the present disclosure, the room temperature refers to 15-30 °C, preferably 20-25 °C.

According to an embodiment of the present disclosure, the mixture is stirred at room temperature for a time period of 1-5 h, e.g., 3 h.

According to an embodiment of the present disclosure, the preparation method for the crystalline form V comprises the following steps:
mixing compound A with acetonitrile according to a mass-to-volume ratio of 1 g to (5-15) mL, stirring the mixture at room temperature until complete dissolution is achieved, and performing filtration and drying *in vacuo* to give the crystalline form V

The present disclosure further provides a preparation method for the above crystalline form VI comprising the following steps:
mixing compound A with a third alcohol solvent, stirring the mixture at room temperature until complete dissolution is achieved, and performing filtration and drying to give the crystalline form VI.

According to an embodiment of the present disclosure, the third alcohol solvent is selected from methanol.

According to an embodiment of the present disclosure, a mass-to-volume ratio of compound A to the third alcohol solvent is 1 g : (5-15) mL, preferably 1 g : (8-12) mL, and exemplarily 1 g : 10 mL.

According to an embodiment of the present disclosure, the room temperature refers to 15-30 °C, preferably 20-25 °C.

According to an embodiment of the present disclosure, the mixture is stirred at room temperature for a time period of 1-5 h, e.g., 3 h.

According to an embodiment of the present disclosure, the preparation method for the crystalline form VI comprises the following steps:
mixing compound A with methanol according to a mass-to-volume ratio of 1 g : (5-15) mL, stirring the mixture at room temperature until complete dissolution is achieved, and performing filtration and drying *in vacuo* to give the crystalline form VI.

The present disclosure further provides a preparation method for the above crystalline form VII comprising the following steps:
mixing compound A with a first organic solvent, stirring the mixture at room temperature until complete dissolution is achieved, and performing filtration and drying to give the crystalline form VII.

According to an embodiment of the present disclosure, the first organic solvent may be selected from one, two or more of butanone, isopropyl acetate, ethanol, and n-butanol, preferably butanone.

According to an embodiment of the present disclosure, a mass-to-volume ratio of compound A to the first organic solvent is 1 g : (5-15) mL, preferably 1 g : (8-12) mL, and exemplarily 1 g : 10 mL.

According to an embodiment of the present disclosure, the room temperature refers to 15-30 °C, preferably 20-25 °C.

According to an embodiment of the present disclosure, the mixture is stirred at room temperature for a time period of 1-5 h, e.g., 3 h.

According to an embodiment of the present disclosure, the preparation method for the crystalline form VII comprises the following steps:
mixing compound A with butanone according to a mass-to-volume ratio of 1 g : (5-15) mL, stirring the mixture at room temperature until complete dissolution is achieved, and performing filtration and drying *in vacuo* to give the crystalline form VII.

The present disclosure further provides another preparation method for the crystalline form VII comprising the following step: heating the crystalline form VI to give the crystalline form VII.

According to an embodiment of the present disclosure, the crystalline form VI is heated to such a temperature that the solvent therein can be completely removed. Preferably, the solvent includes a third alcohol solvent and water. For example, the crystalline form VI is heated to a temperature of no less than 130 °C.

The present disclosure further provides another preparation method for the above crystalline form VII comprising the following steps:
mixing compound A with a fourth alcohol solvent, heating and stirring the system until complete dissolution is achieved, and cooling the system; subsequently, adding an organic acid ester to the system, and concentrating the system *in vacuo* until a volume ratio of the fourth alcohol solvent to the organic acid ester in the system is less than 5%; and supplementing the system with isopropyl acetate, cooling again and then stirring the system, and performing filtration and drying to give the crystalline form VII.

According to an embodiment of the present disclosure, the fourth alcohol solvent may be selected from ethanol and/or n-butanol, preferably ethanol.

According to an embodiment of the present disclosure, the organic acid ester may be selected from isopropyl acetate and/or ethyl acetate, preferably isopropyl acetate.

According to an embodiment of the present disclosure, a mass-to-volume ratio of compound A to the fourth alcohol solvent is 1 g : (2-10) mL, preferably 1 g : (3-8) mL, and exemplarily 1 g : 5 mL.

According to an embodiment of the present disclosure, the system is heated to a temperature of 65-80 °C, preferably 70-75 °C.

According to an embodiment of the present disclosure, the system is heated and stirred for a time period of 0.5-3 h, preferably 1 h.

According to an embodiment of the present disclosure, the system is cooled to a temperature of 40-45 °C.

According to an embodiment of the present disclosure, before the *in vacuo* concentration, the organic acid ester is added to the system in such an amount that a ratio of the volume of the organic acid ester to the mass of compound A is (5-15) mL : 1 g, preferably (7-12) mL : 1 g, and exemplarily 10 mL : 1 g. Preferably, the organic acid ester is added in batches, e.g., in at least two batches, to the system. The organic acid ester is added multiple times to facilitate the removal of the fourth alcohol solvent.

According to an embodiment of the present disclosure, a ratio of the volume of the supplementary organic acid ester to the mass of compound A is (5-15) mL : 1 g, preferably (7-12) mL : 1 g, and exemplarily 8 mL : 1 g.

According to an embodiment of the present disclosure, the system is cooled again to room temperature. Preferably, the room temperature refers to 20-25 °C.

According to an embodiment of the present disclosure, the stirring after the cooling is performed for a time period of 1-5 h, e.g., 3 h.

According to an exemplary embodiment of the present disclosure, the preparation method for the crystalline form VII comprises the following steps:
mixing compound A with ethanol, heating the system to 70-75 °C, stirring the system until complete dissolution is achieved, and cooling the system to 40-45 °C; subsequently, adding isopropyl acetate in batches to the system, and concentrating the system *in vacuo* until a volume ratio of ethanol to isopropyl acetate in the system is less than 5%; and supplementing the system with isopropyl acetate, cooling the system again to 20-25 °C and then stirring the system, and performing filtration and drying *in vacuo* to give the crystalline form VII; wherein
a mass-to-volume ratio of compound A to ethanol is 1 g : (2-10) mL; a ratio of the volume of the supplementary isopropyl acetate to the mass of compound A is (5-15) mL : 1 g.

The present disclosure further provides another preparation method for the above crystalline form VII comprising the following step: mixing and slurrying a mixture of the crystalline form I, the crystalline form III, the crystalline form VII and the crystalline form IX with a second organic solvent to give the crystalline form VII.

Preferably, a mass ratio of the crystalline form I to the crystalline form III to the crystalline form VII to the crystalline form IX is (0.9-1.1):(0.9-1.1): 1:(0.9-1.1).

Preferably, the second organic solvent may be selected from one, two or more of butanone, ethyl acetate, isopropyl acetate, ethanol and n-butanol, preferably butanone or isopropyl acetate.

Preferably, a mass-to-volume ratio of the mixture to the second organic solvent is (15-30) mg : 0.5 mL, e.g., 20 mg : 0.5 mL, 20 mg : 0.4 mL, or 20 mg : 1 mL.

Preferably, the mixture is slurried at a temperature of 15-60 °C, e.g., 20-50 °C.

The present disclosure further provides a method for preserving the crystalline form III or VII, wherein the crystalline form III or VII is placed at a relative humidity level of less than 75% RH, e.g., 70% RH or less.

Preferably, in the method for preserving the crystalline form III or VII, the crystalline form III or VII may be placed at a temperature from room temperature to 60 °C, e.g., 40-60 °C.

The present disclosure further provides a pharmaceutical composition comprising one, two or more of the crystalline forms I, II, III, IV, V, VI, VII and IX of compound A, and optionally a pharmaceutically acceptable excipient.

The present disclosure further provides a formulation comprising one, two or more of the crystalline forms I, II, III, IV, V, VI, VII and IX of compound A, and optionally a pharmaceutically acceptable excipient.

The present disclosure further provides use of the above crystalline forms I, II, III, IV, V, VI, VII and/or IX of compound A, or the pharmaceutical composition, in preparing a medicament for preventing and/or treating an IRAK-mediated disease or condition.

According to an embodiment of the present disclosure, the IRAK-mediated disease or condition is selected from tumors, gout, systemic lupus erythematosus, multiple sclerosis, metabolic syndrome, atherosclerosis, myocardial infarction, sepsis, inflammatory bowel disease, asthma, allergy, and the like.

The present disclosure further provides use of the above crystalline forms I, II, III, IV, V, VI, VII and/or IX of compound A, or the pharmaceutical composition, in preparing a medicament for preventing and/or treating a disease or condition associated with interleukin-1 receptor associated kinases.

The present disclosure further provides a method for preventing and/or treating an IRAK-mediated disease or condition comprising administering to a subject in need thereof a therapeutically effective amount of the above crystalline forms I, II, III, IV, V, VI, VII and/or IX of compound A, or the pharmaceutical composition, or the formulation.

In some embodiments, the IRAK is IRAK4-associated kinase.

The present disclosure further provides a method for preventing and/or treating an interleukin-1 receptor-associated disease comprising administering to a subject in need thereof a therapeutically effective amount of the above crystalline forms I, II, III, IV, V, VI, VII and/or IX of compound A, or the pharmaceutical composition, or the formulation.

According to an embodiment of the present disclosure, the disease or condition associated with interleukin-1 receptor-associated kinase is selected from tumors, gout, systemic lupus erythematosus, multiple sclerosis, metabolic syndrome, atherosclerosis, myocardial infarction, sepsis, inflammatory bowel disease, asthma, rheumatoid arthritis, septicemia, autoimmune disease, allergy, and the like.

The methods of the present disclosure may include administering one, two or more crystalline forms of compound A of the present disclosure alone, and administering one, two or more crystalline forms of compound A of the present disclosure in combination with one, two or more other chemotherapeutic agents. Multiple drugs may be administered simultaneously or successively.

### Beneficial Effects of Present Disclosure

1) The present disclosure provides a polymorph of compound A and a preparation method therefor, wherein the preparation method for the polymorph features simplicity of the process, ease of implementation, mild conditions for reaction, and high product yields. Moreover, multiple purification processes are not necessary, and the operation is safe and environment-friendly, favoring industrial production of polymorphs.
2) The polymorph prepared by the present disclosure has good stability and can be stably stored at high temperature and low relative humidity. For example, the crystalline forms III and VII are physically and chemically stable after being let stand at 60 °C (sealed) for 7 days, and are chemically stable after being let stand at 40 °C/75% RH (open) for 7 days;the crystalline forms III and VII are physically stable (the purity, color, appearance, etc.) at 70% RH or less.

Moreover, the crystalline forms of the present disclosure have good fluidity, and are easy to crush and to use for preparing a pharmaceutical composition. Lastly, the polymorph prepared by the present disclosure has high purity and few impurities.

### Definitions and Description

Although the terms and phrases used herein have general meanings known to those skilled in the art, they are still illustrated and explained in detail herein. The meanings of the terms and phrases mentioned described in the present disclosure shall prevail in the event of any inconsistency with those well known.

The polymorphs of compound A of the present disclosure include non-solvate (anhydrate) and solvate crystalline forms of compound A.

The characteristic peaks in X-ray powder diffraction patterns of the polymorphs of compound A of the present disclosure are expressed in terms of 2θ angles, wherein "±0.20°" is an allowed measurement error range.

The polymorphs of compound A of the present disclosure can be used in combination with other active ingredients, provided that they do not produce other adverse effects, such as allergy.

As used in the present disclosure, the term "composition" is intended to encompass a product comprising specified ingredients in specified amounts, as well as any product which results, directly or indirectly, from combination of the specified ingredients in the specified amounts.

The polymorphs of compound A of the present disclosure can be prepared into suitable pharmaceutical compositions using known pharmaceutical carriers by those skilled in the art. The pharmaceutical compositions may be specifically formulated in solid or liquid form for oral administration, for parenteral injection or for rectal administration. The pharmaceutical compositions can be formulated in a variety of dosage forms for ease of administration, e.g., oral formulations (e.g., tablets, capsules, solutions or suspensions), injectable formulations (e.g., injectable solutions or suspensions, or injectable dry powders, which can be used immediately after addition of a pharmaceutical vehicle before injection).

As used herein, the term "therapeutically and/or prophylactically effective amount" refers to an amount of a drug or pharmaceutical formulation that elicits the biological or medical response in a tissue, system, animal or human that is being sought by a researcher, veterinarian, medical doctor or other people.

When used for the above therapeutic and/or prophylactic purposes, the total daily amount of the polymorphs of compound A and the pharmaceutical compositions of the present disclosure will be determined by an attending physician within the scope of sound medical judgment. For any particular patient, the particular therapeutically effective dose level will depend upon a variety of factors including the disorder being treated and the severity of the disorder, the activity of a particular compound employed, the particular composition employed, the age, body weight, general health, sex, and diet of the patient, the time of administration, route of administration and excretion rate of the particular compound employed, the duration of the treatment, the drugs used in combination or simultaneously with the particular compound employed, and similar factors well known in the medical arts. For example, it is known in the art to start a dose of a compound at a level below that required to achieve a desired therapeutic effect and then gradually increase the dose until the desired therapeutic effect is achieved.

### BRIEF DESCRIPTION OF THE DRAWINGS

FIG. 1 shows an XRPD pattern of the crystalline form I.
FIG. 2 shows a PLM image of the crystalline form I (scale bar: 20 µm).
FIG. 3 shows a DSC-TGA pattern of the crystalline form I.
FIG. 4 shows XRPD patterns of the crystalline forms II and III.
FIG. 5 shows a PLM image of the crystalline form II (scale bar: 10 µm).
FIG. 6 shows a PLM image of the crystalline form III (scale bar: 2.5 µm).
FIG. 7 shows a DSC-TGA pattern of the crystalline form II.
FIG. 8 shows a DSC-TGA pattern of the crystalline form III.
FIG. 9 shows a DVS pattern of the crystalline form III.
FIG. 10 shows XRPD patterns of the crystalline form III for comparison before and after DVS tests.
FIG. 11 shows an XRPD pattern of the crystalline form IV
FIG. 12 shows a PLM image of the crystalline form IV (scale bar: 10 µm).
FIG. 13 shows a DSC-TGA pattern of the crystalline form IV
FIG. 14 shows an XRPD pattern of the crystalline form V
FIG. 15 shows a PLM image of the crystalline form V (scale bar: 10 µm).
FIG. 16 shows a DSC-TGA pattern of the crystalline form V
FIG. 17 shows an XRPD pattern of the crystalline form IX.
FIG. 18 shows a PLM image of the crystalline form IX (scale bar: 5 µm).
FIG. 19 shows a DSC-TGA pattern of the crystalline form IX.
FIG. 20 shows an XRPD pattern of the crystalline form VI.
FIG. 21 shows a PLM image of the crystalline form VI (scale bar: 2.5 µm).
FIG. 22 shows a DSC-TGA pattern of the crystalline form VI.
FIG. 23 shows an XRPD pattern of the crystalline form VII.
FIG. 24 shows a PLM image of the crystalline form VII (scale bar: 5 µm).
FIG. 25 shows a DSC-TGA pattern of the crystalline form VII.
FIG. 26 shows a DVS pattern of the crystalline form VII.
FIG. 27 shows XRPD patterns of the crystalline form VII for comparison before and after DVS tests.
FIG. 28 shows an overlay of XRPD patterns of the stability samples.
FIG. 29 shows an overlay of XRPD patterns of the crystalline form III sample in the tests for effects of humidity.
FIG. 30 shows an overlay of XRPD patterns of the crystalline form VII sample in the tests for effects of humidity.
FIG. 31 shows a DVS dynamic curve in the tests for effects of humidity.
FIG. 32 shows an overlay of XRPD patterns of the dried crystalline form III sample in the humidity tests.
FIG. 33 shows an overlay of XRPD patterns of the dried crystalline form VII sample in the humidity tests.
FIG. 34 shows the results of the solubility tests.
FIG. 35 shows an overlay of XRPD patterns of the samples after solubility tests.

### DETAILED DESCRIPTION

The technical scheme of the present disclosure will be further illustrated in detail with reference to the following specific examples. It should be understood that the following examples are merely exemplary illustration and explanation of the present disclosure, and should not be construed as limiting the protection scope of the present disclosure. All techniques implemented based on the aforementioned content of the present disclosure are encompassed within the protection scope of the present disclosure.

Unless otherwise stated, the starting materials and reagents used in the following examples are all commercially available products or can be prepared by known methods.

### Synthesis of compound A

### Reaction scheme:

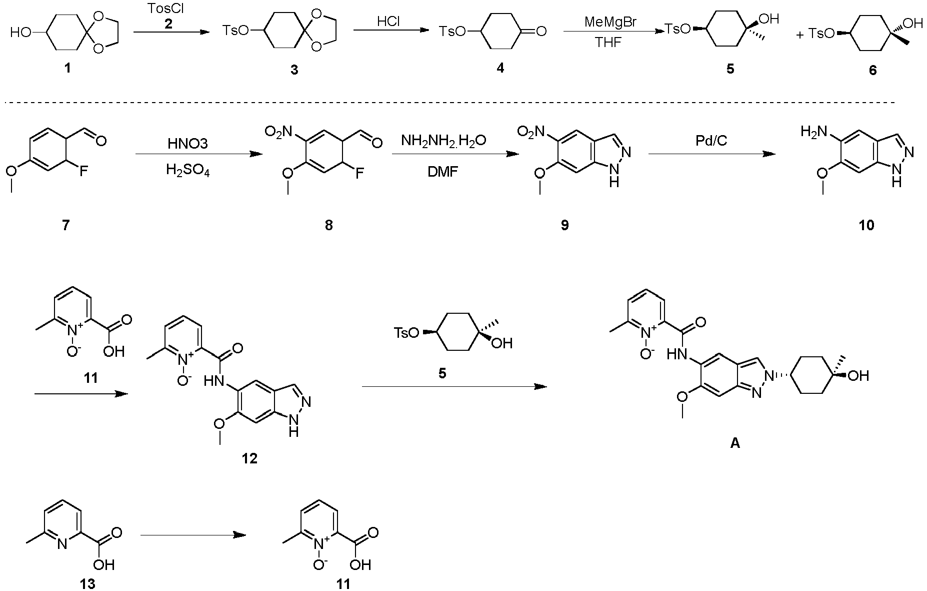

### (1) Synthesis of compound 3

DMAP (42.5 g), compound 2 (63.4 g) and triethylamine (63.9 g) were added sequentially to a solution of compound 1 (50 g) in dichloromethane (500 mL) at 15 °C. The reaction mixture was stirred at 25 °C for 18 h. To the reaction mixture was added dichloromethane (200 mL), and the resulting mixture was washed with water (300 mL × 2) and 1 M hydrochloric acid (300 mL × 3). The organic phase was dried over anhydrous sodium sulfate, filtered and concentrated under reduced pressure to give a yellow solid (compound 3, 98 g, 99% yield).

### (2) Synthesis of compound 4

1 M hydrochloric acid (300 mL) was added to a solution of compound 3 (50 g) in tetrahydrofuran (300 mL) at 15 °C. The reaction mixture was stirred at 25 °C for 20 h. The reaction mixture was cooled to 0 °C, adjusted to pH = 9 with 1 M sodium hydroxide solution and extracted with ethyl acetate (200 mL × 3). The extracts were washed with saturated sodium chloride solution (300 mL), dried over anhydrous sodium sulfate, filtered and concentrated under reduced pressure. The residue was slurried with petroleum ether (150 mL) to give a white solid (compound 4, 39 g, 91% yield).

### (3) Synthesis of compounds 5 & 6

A solution of compound **4** (34.5 g) in tetrahydrofuran (200 mL) was dropwise added to a solution of methylmagnesium bromide (85.8 mL) in tetrahydrofuran (500 mL) at -40 °C. The reaction mixture was stirred at -40 °C for 4 h, then quenched with saturated ammonium chloride solution (100 mL) and extracted with ethyl acetate (500 mL × 3). The extracts were washed with saturated saline (300 mL), dried over anhydrous sodium sulfate, filtered and concentrated under reduced pressure. The residue was purified by silica gel column chromatography (petroleum ether:ethyl acetate = 5:1) to give compound **5** in the form of a colorless oil (4.3 g, 10% yield), compound **6** in the form of a colorless oil (7.0 g, 17% yield) and a mixture (12 g).

### Compound 5

¹H NMR (400 MHz, CDCl₃): δ 7.79 (d, *J* = 8.0 Hz, 2H), 7.32 (d, *J* = 8.4 Hz, 2H), 4.52-4.41 (m, 1H), 2.44 (s, 3H), 1.95-1.80 (m, 2H), 1.77-1.61 (m, 4H), 1.46-1.35 (m, 2H), 1.19 (s, 3H).

### Compound 6

¹H NMR (400 MHz, CDCl₃): δ 7.79 (d, *J* = 8.4 Hz, 2H), 7.33 (d, *J* = 8.0 Hz, 2H), 4.74-4.64 (m, 1H), 2.44 (s, 3H), 1.92-1.79 (m, 2H), 1.77-1.62 (m, 4H), 1.49-1.38 (m, 2H),1.23 (s, 3H).

### (4) Synthesis of compound 8

A mixture of nitric acid (1.6 mL, 70%) and concentrated sulfuric acid (1.6 mL, 98%) was added dropwise to a solution of compound **7** (2.0 g) in concentrated sulfuric acid (12 mL, 98%) at -15 °C. After the addition was completed, the reaction mixture was stirred at -15 °C for 2 h and then slowly poured into ice water. The resulting mixture was stirred for 5 min and subjected to suction filtration. The filter cake was washed with water, collected and dried under reduced pressure to give a yellow solid (compound 8, 2.5 g, 97% yield).

### (5) Synthesis of compound 9

Hydrazine hydrate (2.4 mL, 98%) was added to a solution of compound **8** (2.0 g) in DMF (20 mL) at room temperature. After the addition was completed, the reaction mixture was heated to 120 °C, stirred for 16 h, cooled to room temperature and slowly poured into ice water. The resulting mixture was stirred and subjected to suction filtration. The filter cake was washed with water, collected and dried under reduced pressure to give a yellow solid (compound 9, 1.3 g, 67% yield).

### (6) Synthesis of compound 10

Compound **9** (12.4 g) and palladium on carbon (7 g, 10%) were added sequentially to ethyl acetate (400 mL) at 15 °C. After the addition was completed, the reaction mixture was stirred at 15 °C in a hydrogen atmosphere for 18 h. The palladium on carbon in the reaction mixture was filtered out, and the filtrate was concentrated to dryness to give a white solid (compound 10, 10.4 g, 99% yield).

### (7) Synthesis of compound 12

EDCI.HCl (2.6 g) was added to a solution of compound **10** (1.5 g) and compound **11** (1.4 g) in Py (15 mL) at 25 °C. The reaction mixture was stirred at 25 °C for 16 h. The reaction mixture was concentrated to dryness, and the residue was slurried with MeOH/H₂O = 20 mL/20 mL to give a yellow solid (compound 12, 1.3 g, 48% yield).

### (8) Synthesis of compound A

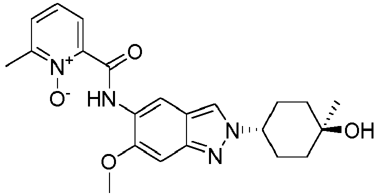

Cesium carbonate (985 mg) was added to a solution of compound **12** (300 mg) and compound **5** (344 mg) in DMF (5 mL) at 25 °C. The reaction mixture was stirred at 90 °C for 16 h. The reaction mixture was added to water (30 mL), and the resulting mixture was extracted with ethyl acetate (10 mL × 3). The organic phase was concentrated under reduced pressure. The residue was purified by preparative high performance liquid chromatography (CH₃CN:H₂O (0.1% NH₄HCO₃) = 15-45%, UV: 214 nm, flowrate: 15 mL/min) to give a yellow solid (compound A, 70 mg, 17% yield).

¹H NMR (400 MHz, DMSO-*d*₆): δ 14.16 (s, 1H), 8.78 (s, 1H), 8.34 (s, 1H), 8.32-8.30 (m, 1H), 7.77 (d, *J* = 7.6 Hz, 1H), 7.58 (t, *J* = 8.0 Hz, 1H), 7.13 (s, 1H), 4.45 (s, 1H), 4.43-4.40 (m, 1H), 3.95 (s, 3H), 2.53 (s, 3H), 2.09-2.00 (m, 4H), 1.68-1.58 (m, 4H), 1.22 (s, 3H). LCMS: Rt = 3.646 min, [M+H]⁺ = 411.1.

### (9) Synthesis of compound 11

*m*-CPBA (25 g) was added to a solution of compound **13** (10 g) in DCM (200 mL) at 25 °C. The reaction mixture was stirred at 25 °C for 16 h. The reaction mixture was filtered, and the filtrate was quenched with a saturated solution prepared from sodium sulfite (15.6 g). The resulting mixture was stirred for 2 h and extracted. The aqueous phase was adjusted to pH < 7 with dilute hydrochloric acid, and DCM (50 mL × 3) was added for extraction. The organic phases were combined and concentrated, and the residue was slurried with EA (300 mL) to give a white solid (compound 11, 10.1 g, 90% yield).

In the following examples, XRPD tests were carried out using a PIXceI^{1D} detector under the following conditions: PANalytical EMPYREAN.

DVS tests were carried out using a dynamic vapor sorption instrument (Vsorp-Enhanced, proUmid) under the following conditions: adding a sufficient amount of a sample to the Vsorp-Enhanced instrument for simulation of dynamic vapor sorption, recording changes in weight at 25 °C with different humidity equilibrium levels, and subjecting the post-DVS test sample to an XRPD test.

### Example 1

### Preparation of crystalline form I:

Compound A (1 g) was added to ethanol/methyl *tert*-butyl ether (15 mL/5 mL). The mixture was heated to 60 °C, stirred for 3 h, cooled to 0-10 °C, and filtered. The filter cake was dried *in vacuo* to give the crystalline form I (0.85 g) with 99% or more purity.

The crystalline form I was characterized by XRPD, PLM, DSC and TGA. The crystalline form I is an anhydrate. The locations and intensities of XRPD characteristic peaks are shown in Table 1, and the XRPD pattern is shown in FIG. 1. The PLM image shows that the sample is a crystal with irregular morphology and a size of 20 µm or less (FIG. 2). The TGA test of the sample shows a weight loss of 1.2% from 140 to 200 °C (FIG. 3), which corresponds to 0.9% ethanol and 0.3% methyl *tert*-butyl ether remaining in the sample as indicated by nuclear magnetic resonance test. The DSC test of the sample shows only one endothermic peak with an initial temperature of 191 °C (FIG. 3).

In the XRPD pattern of the crystalline form I expressed in terms of 2θ angles, the 2θ values are shown in Table 1:

**Table 1. XRPD characteristic peaks of crystalline form I**

| **Peak number** | **2θ [ ° ]** | **d [ Å ]** | **Relative intensity (%)** | **Peak number** | **2θ [ ° ]** | **d [ Å ]** | **Relative intensity (%)** |
|---|---|---|---|---|---|---|---|
| 1 | 4.541 | 19.4422 | 0.5 | 25 | 24.929 | 3.5689 | 5.2 |
| 2 | 6.02 | 14.6683 | 11.9 | 26 | 25.175 | 3.5345 | 4.3 |
| 3 | 6.925 | 12.754 | 2.2 | 27 | 25.597 | 3.4772 | 1.8 |
| 4 | 7.557 | 11.6885 | 0.5 | 28 | 26.214 | 3.3967 | 4.7 |
| 5 | 10.471 | 8.4413 | 2.9 | 29 | 27.029 | 3.2962 | 0.9 |
| 6 | 10.916 | 8.098 | 4.4 | 30 | 27.765 | 3.2105 | 9.6 |
| 7 | 11.851 | 7.4616 | 32.6 | 31 | 28.528 | 3.1262 | 0.4 |
| 8 | 12.126 | 7.2928 | 100 | 32 | 29.103 | 3.0658 | 1 |
| 9 | 13.674 | 6.4703 | 3 | 33 | 29.602 | 3.0152 | 4.2 |
| 10 | 14.963 | 5.9157 | 2.2 | 34 | 30.259 | 2.9513 | 0.6 |
| 11 | 15.237 | 5.8102 | 6.2 | 35 | 30.757 | 2.9046 | 1.3 |
| 12 | 15.868 | 5.5803 | 35.1 | 36 | 30.98 | 2.8842 | 1.3 |
| 13 | 16.261 | 5.4463 | 13.7 | 37 | 31.78 | 2.8133 | 1 |
| 14 | 16.577 | 5.3434 | 36.9 | 38 | 32.49 | 2.7535 | 3.3 |
| 15 | 17.404 | 5.0913 | 8 | 39 | 32.977 | 2.7139 | 1.9 |
| 16 | 17.68 | 5.0124 | 17.2 | 40 | 33.354 | 2.6841 | 0.9 |
| 17 | 18.337 | 4.8344 | 9.9 | 41 | 34.172 | 2.6217 | 0.6 |
| 18 | 19.15 | 4.6308 | 3.8 | 42 | 34.67 | 2.5852 | 1 |
| 19 | 20.989 | 4.229 | 13.2 | 43 | 35.182 | 2.5488 | 1.6 |
| 20 | 21.593 | 4.1121 | 4.1 | 44 | 35.563 | 2.5223 | 0.2 |
| 21 | 22.446 | 3.9577 | 1.3 | 45 | 37.046 | 2.4247 | 1 |
| 22 | 22.681 | 3.9172 | 2.2 | 46 | 37.874 | 2.3735 | 1.4 |
| 23 | 23.457 | 3.7894 | 17.3 | 47 | 38.752 | 2.3217 | 0.8 |
| 24 | 23.996 | 3.7055 | 38.4 | | | | |

### Example 2

### Preparation of crystalline forms II and III:

Compound A (1 g) was added to toluene (15 mL). The mixture was stirred at 20-25 °C for 3 h, and filtered. The filter cake was the crystalline form II with 99% or more purity.

The crystalline form II obtained was dried *in vacuo* at 50-60 °C to give the crystalline form III (0.90 g) with 99% or more purity.

The crystalline form II was characterized by XRPD, PLM, DSC and TGA. The locations and intensities of XRPD characteristic peaks are shown in Table 2, and the XRPD pattern is shown in FIG. 4. The PLM image (FIG. 5) shows that the sample is a crystal with irregular morphology, a particle size < 10 µm and high crystallinity. The TGA test of the sample shows a weight loss of 9.6% from 100 to 160 °C (FIG. 7), which corresponds to 9.5% toluene remaining in the sample as indicated by nuclear magnetic resonance test. Therefore, the crystalline form II is a toluene solvate. The DSC of crystalline form II shows a first endothermic peak at a peak temperature raised to near 136.92 °C, and a second endothermic peak at a peak temperature raised to near 189.27 °C. After the solvent was removed, the crystalline form II was transformed into the crystalline form III.

The crystalline form III was characterized by XRPD, PLM, DSC, TGA and DVS. The locations and intensities of XRPD characteristic peaks are shown in Table 3, and the XRPD pattern is shown in FIG. 4. The PLM image (FIG. 6) shows that the sample is a crystal with irregular morphology and a particle size < 5 µm. The crystallinity is high. The TGA of the sample shows almost no weight loss below 180 °C (FIG. 8). The DSC pattern shows an initial melting point of the sample of about 187 °C (FIG. 8). The DVS results (FIG. 9) show slight hygroscopicity at only 0.22% within a range of 0-80% RH. However, the crystalline form III was transformed into a crystal mixture of the crystalline forms III and IV after the DVS test (FIG. 10).

In the XRPD patterns of the crystalline forms II and III expressed in terms of 2θ angles, the 2θ values of the two are shown in Tables 2 and 3, respectively:

**Table 2. XRPD characteristic peaks of crystalline form II**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 5.205 | 16.9637 | 1.8 | 28 | 25.743 | 3.4578 | 3.5 |
| 2 | 9.799 | 9.0185 | 6 | 29 | 26.097 | 3.4117 | 2 |
| 3 | 10.537 | 8.3888 | 100 | 30 | 26.463 | 3.3653 | 1 |
| 4 | 11.651 | 7.5888 | 8.1 | 31 | 26.782 | 3.326 | 0.7 |
| 5 | 12.178 | 7.2619 | 5.7 | 32 | 27.317 | 3.262 | 30.9 |
| 6 | 13.492 | 6.5575 | 60.8 | 33 | 27.845 | 3.2014 | 0.3 |
| 7 | 14.028 | 6.3079 | 19 | 34 | 28.315 | 3.1493 | 7.4 |
| 8 | 14.317 | 6.1813 | 0.7 | 35 | 28.563 | 3.1226 | 5.8 |
| 9 | 15.055 | 5.8799 | 3.5 | 36 | 29.353 | 3.0403 | 5.7 |
| 10 | 15.211 | 5.8201 | 4.6 | 37 | 29.654 | 3.0101 | 2.6 |
| 11 | 15.776 | 5.6127 | 5.3 | 38 | 30.351 | 2.9426 | 6.3 |
| 12 | 16.287 | 5.4376 | 9.8 | 39 | 30.707 | 2.9092 | 1.5 |
| 13 | 17.156 | 5.1643 | 19.1 | 40 | 31.022 | 2.8804 | 2 |
| 14 | 17.509 | 5.061 | 52.1 | 41 | 31.374 | 2.8489 | 3.3 |
| 15 | 17.719 | 5.0014 | 53 | 42 | 31.545 | 2.8338 | 3.5 |
| 16 | 18.427 | 4.8107 | 3.8 | 43 | 32.95 | 2.7161 | 4.8 |
| 17 | 19.584 | 4.5292 | 15.5 | 44 | 33.488 | 2.6737 | 1.3 |
| 18 | 19.767 | 4.4877 | 18.3 | 45 | 34.198 | 2.6198 | 0.8 |
| 19 | 20.358 | 4.3587 | 17.6 | 46 | 34.579 | 2.5918 | 2.3 |
| 20 | 20.976 | 4.2317 | 27.9 | 47 | 35.222 | 2.5459 | 0.8 |
| 21 | 21.933 | 4.049 | 2.1 | 48 | 35.63 | 2.5177 | 2.3 |
| 22 | 22.278 | 3.9871 | 1 | 49 | 36.469 | 2.4617 | 1.6 |
| 23 | 22.853 | 3.8882 | 3.6 | 50 | 36.705 | 2.4464 | 2.8 |
| 24 | 23.065 | 3.8528 | 1.3 | 51 | 37.376 | 2.404 | 2.9 |
| 25 | 23.667 | 3.7563 | 68.6 | 52 | 37.929 | 2.3702 | 0.8 |
| 26 | 24.533 | 3.6256 | 3 | 53 | 38.567 | 2.3325 | 1.1 |
| 27 | 25.354 | 3.51 | 0.2 | 54 | 39.015 | 2.3067 | 0.6 |

**Table 3. XRPD characteristic peaks of crystalline form III**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 6.021 | 14.6671 | 5.2 | 22 | 26.083 | 3.4135 | 37.3 |
| 2 | 10.879 | 8.1259 | 8.2 | 23 | 26.688 | 3.3375 | 1.3 |
| 3 | 12.152 | 7.2773 | 77.4 | 24 | 27.106 | 3.287 | 1 |
| 4 | 12.978 | 6.8157 | 3.6 | 25 | 27.83 | 3.203 | 5.8 |
| 5 | 15.04 | 5.8857 | 20.5 | 26 | 28.262 | 3.1551 | 0.2 |
| 6 | 15.986 | 5.5397 | 47.8 | 27 | 29.314 | 3.0442 | 9.1 |
| 7 | 16.617 | 5.3306 | 34.2 | 28 | 29.824 | 2.9933 | 3.2 |
| 8 | 17.141 | 5.1688 | 27 | 29 | 30.429 | 2.9351 | 3.6 |
| 9 | 18.323 | 4.8378 | 1.5 | 30 | 30.818 | 2.899 | 1.5 |
| 10 | 18.742 | 4.7306 | 13.7 | 31 | 31.926 | 2.8009 | 4.9 |
| 11 | 20.068 | 4.4209 | 8.7 | 32 | 32.373 | 2.7632 | 3.1 |
| 12 | 20.449 | 4.3394 | 3.5 | 33 | 32.583 | 2.7459 | 2.3 |
| 13 | 20.765 | 4.2741 | 3.2 | 34 | 33.186 | 2.6973 | 1.4 |
| 14 | 21.092 | 4.2086 | 21.8 | 35 | 33.777 | 2.6515 | 2.5 |
| 15 | 21.645 | 4.1024 | 2.2 | 36 | 34.392 | 2.6054 | 0.6 |
| 16 | 22.262 | 3.99 | 5.1 | 37 | 35.418 | 2.5323 | 2.4 |
| 17 | 22.97 | 3.8685 | 0.4 | 38 | 36.023 | 2.4912 | 1.7 |
| 18 | 23.51 | 3.781 | 19.6 | 39 | 36.6 | 2.4532 | 2 |
| 19 | 24.048 | 3.6976 | 100 | 40 | 38.138 | 2.3577 | 4 |
| 20 | 24.323 | 3.6564 | 50.8 | 41 | 38.806 | 2.3186 | 4.3 |
| 21 | 24.598 | 3.6161 | 8.3 | 42 | 39.306 | 2.2903 | 2 |

### Example 3

### Preparation of crystalline form IV:

Compound A (1 g) was added to isopropanol/water (5 mL/5 mL). The mixture was stirred at 20-25 °C for 3 h, and filtered. The filter cake was dried *in vacuo* to give the crystalline form IV (0.90 g) with 99% or more purity.

After being heated to 100 °C and completely dehydrated, the crystalline form IV was transformed into the crystalline form I.

The crystalline form IV was characterized by XRPD, PLM, DSC and TGA. The locations and intensities of XRPD characteristic peaks are shown in Table 4, and the XRPD pattern is shown in FIG. 11. The PLM image (FIG. 12) shows that the sample is a crystal with irregular morphology, a particle size < 10 µm and high crystallinity. The DSC pattern shows multiple endothermic peaks (FIG. 13), indicating that the sample may undergo polymorphic transition during heating. The TGA of the sample shows a weight loss of 16.9% from room temperature to 150 °C (FIG. 13), with a water content of 4.2wt%. Crystalline form IV is a monohydrate of compound A. After being completely dehydrated, the crystalline form IV was transformed into the crystalline form I (FIG. 11).

In the XRPD pattern of the crystalline form IV expressed in terms of 2θ angles, the 2θ values are shown in Table 4:

**Table 4. XRPD characteristic peaks of crystalline form IV**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 4.584 | 19.2594 | 1.3 | 24 | 23.916 | 3.7177 | 4.5 |
| 2 | 5.377 | 16.4225 | 69.2 | 25 | 24.677 | 3.6048 | 2.8 |
| 3 | 6.677 | 13.2268 | 28.9 | 26 | 25.529 | 3.4863 | 10.8 |
| 4 | 9.762 | 9.0531 | 27.6 | 27 | 25.952 | 3.4304 | 8.1 |
| 5 | 10.024 | 8.817 | 52 | 28 | 26.426 | 3.37 | 0.7 |
| 6 | 10.736 | 8.2335 | 7.8 | 29 | 26.959 | 3.3045 | 3 |
| 7 | 12.442 | 7.1083 | 2.7 | 30 | 27.163 | 3.2802 | 4.4 |
| 8 | 13.425 | 6.5901 | 3.2 | 31 | 27.476 | 3.2435 | 11.6 |
| 9 | 13.753 | 6.4335 | 6.4 | 32 | 27.815 | 3.2048 | 10.6 |
| 10 | 14.818 | 5.9735 | 100 | 33 | 28.633 | 3.115 | 2 |
| 11 | 15.355 | 5.7658 | 5.1 | 34 | 29.655 | 3.01 | 12.4 |
| 12 | 16.183 | 5.4725 | 7.9 | 35 | 31.032 | 2.8795 | 5.6 |
| 13 | 16.604 | 5.3347 | 9.3 | 36 | 31.776 | 2.8138 | 1.2 |
| 14 | 16.881 | 5.2478 | 2.9 | 37 | 32.752 | 2.7321 | 1.1 |
| 15 | 17.494 | 5.0654 | 2.9 | 38 | 33.988 | 2.6355 | 0.7 |
| 16 | 18.638 | 4.7569 | 7.7 | 39 | 35.007 | 2.5611 | 2.8 |
| 17 | 19.689 | 4.5053 | 63.4 | 40 | 35.351 | 2.537 | 2 |
| 18 | 20.134 | 4.4067 | 11 | 41 | 36.377 | 2.4677 | 2.2 |
| 19 | 20.519 | 4.3249 | 0.9 | 42 | 37.479 | 2.3977 | 3 |
| 20 | 21.414 | 4.1461 | 3 | 43 | 38.361 | 2.3445 | 2.2 |
| 21 | 21.672 | 4.0972 | 2.9 | 44 | 38.465 | 2.3384 | 2.2 |
| 22 | 22.957 | 3.8707 | 7.2 | 45 | 39.566 | 2.2758 | 2.1 |
| 23 | 23.372 | 3.803 | 3.7 | | | | |

### Example 4

### Preparation of crystalline forms V and IX:

Compound A (1 g) was added to acetonitrile (10 mL). The mixture was stirred at 20-25 °C for 3 h, and filtered. The filter cake was dried *in vacuo* to give the crystalline form V (0.90 g) with 99% or more purity.

After being heated to 150 °C, the crystalline form V was transformed into the crystalline form IX with 99% or more purity.

The crystalline form V was characterized by XRPD, PLM, DSC and TGA. The locations and intensities of XRPD characteristic peaks are shown in Table 5, and the XRPD pattern is shown in FIG. 14. The PLM image (FIG. 15) shows that the sample is a crystal with irregular morphology and a particle size < 10 µm. The crystallinity is high. The DSC (FIG. 16) of the sample shows two endothermic peaks (a second endothermic peak at a peak temperature raised to near 192.24 °C, and a third endothermic peak at a peak temperature raised to near 218.33 °C) after the solvent in the sample was removed (a first endothermic peak at a peak temperature raised to near 135.05 °C), suggesting possible polymorphic transition during heating. The TGA test (FIG. 16) of the sample shows a weight loss of 6.4% between 70 and 150 °C, which corresponds to 5.1% acetonitrile remaining in the sample as indicated by nuclear magnetic resonance test. Therefore, the crystalline form V is an acetonitrile solvate. After the solvent was removed, the crystalline form V was transformed into the crystalline form IX (FIGs. 14 and 17).

The crystalline form IX was characterized by XRPD, PLM, DSC and TGA. The locations and intensities of XRPD characteristic peaks are shown in Table 6, and the XRPD pattern is shown in FIG. 17. The PLM image (FIG. 18) shows that the sample is a crystal with irregular morphology and a particle size < 5 µm. The crystallinity is high. The DSC shows multiple endothermic peaks (a first endothermic peak at a peak temperature raised to near 192.04 °C, a second endothermic peak at a peak temperature raised to near 201.20 °C, and a third endothermic peak at a peak temperature raised to near 217.55 °C), suggesting possible polymorphic transition during heating (FIG. 19), indicating that the sample may undergo polymorphic transition during heating. The TGA of the sample shows almost no weight loss below 180 °C (FIG. 19).

In the XRPD patterns of the crystalline forms V and IX expressed in terms of 2θ angles, the 2θ values of the two are shown in Tables 5 and 6, respectively:

**Table 5. XRPD characteristic peaks of crystalline form V**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 5.547 | 15.9199 | 0.6 | 15 | 25.649 | 3.4702 | 18.5 |
| 2 | 7.11 | 12.4232 | 98 | 16 | 26.371 | 3.3768 | 1.5 |
| 3 | 8.514 | 10.377 | 4.2 | 17 | 27.582 | 3.2313 | 1.6 |
| 4 | 9.617 | 9.1895 | 26.1 | 18 | 28.447 | 3.135 | 2.3 |
| 5 | 11.234 | 7.87 | 16.2 | 19 | 29.246 | 3.0512 | 1.5 |
| 6 | 12.556 | 7.0443 | 0.3 | 20 | 30.219 | 2.955 | 2.6 |
| 7 | 14.069 | 6.2897 | 43.3 | 21 | 32.477 | 2.7546 | 4.1 |
| 8 | 16.038 | 5.5216 | 100 | 22 | 33.462 | 2.6757 | 0.9 |
| 9 | 17.587 | 5.0387 | 1.1 | 23 | 34.307 | 2.6117 | 0.6 |
| 10 | 19.229 | 4.6119 | 16.9 | 24 | 36.429 | 2.4643 | 3.7 |
| 11 | 21.592 | 4.1122 | 26 | 25 | 37.624 | 2.3888 | 1.2 |
| 12 | 22.051 | 4.0278 | 5.8 | 26 | 38.187 | 2.3548 | 0.5 |
| 13 | 22.985 | 3.866 | 8.1 | 27 | 39.331 | 2.2889 | 3.7 |
| 14 | 23.602 | 3.7664 | 2.1 | 28 | 39.632 | 2.2722 | 3.4 |

**Table 6. XRPD characteristic peaks of crystalline form IX**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 5.482 | 16.1066 | 1 | 19 | 23.641 | 3.7603 | 11.6 |
| 2 | 8.265 | 10.6892 | 43.8 | 20 | 24.757 | 3.5932 | 12.5 |
| 3 | 9.329 | 9.4723 | 35.9 | 21 | 25.086 | 3.5469 | 12.2 |
| 4 | 10.524 | 8.3989 | 9.6 | 22 | 25.769 | 3.4544 | 0.2 |
| 5 | 11.075 | 7.9821 | 14.4 | 23 | 26.097 | 3.4116 | 7.5 |
| 6 | 12.953 | 6.829 | 100 | 24 | 26.871 | 3.3152 | 12.8 |
| 7 | 14.686 | 6.0267 | 3.5 | 25 | 27.419 | 3.2501 | 0.3 |
| 8 | 16.814 | 5.2687 | 95.7 | 26 | 27.75 | 3.2122 | 1 |
| 9 | 17.666 | 5.0164 | 5.7 | 27 | 28.183 | 3.1637 | 3.2 |
| 10 | 18.296 | 4.8451 | 1 | 28 | 29.169 | 3.059 | 10.6 |
| 11 | 18.795 | 4.7175 | 8.7 | 29 | 29.417 | 3.0338 | 10.7 |
| 12 | 19.423 | 4.5662 | 1.2 | 30 | 30.784 | 2.9021 | 1.1 |
| 13 | 19.834 | 4.4726 | 1 | 31 | 31.532 | 2.835 | 0.3 |
| 14 | 20.726 | 4.2821 | 16.7 | 32 | 33.448 | 2.6768 | 2.5 |
| 15 | 21.015 | 4.2238 | 24.7 | 33 | 33.711 | 2.6565 | 4.5 |
| 16 | 22.051 | 4.0276 | 2 | 34 | 35.81 | 2.5054 | 1.3 |
| 17 | 22.471 | 3.9533 | 7.5 | 35 | 37.164 | 2.4172 | 1 |
| 18 | 23.274 | 3.8188 | 13.5 | 36 | 38.175 | 2.3555 | 1.4 |

### Example 5

### Preparation of crystalline form VI:

Compound A (1 g) was added to methanol (10 mL). The mixture was stirred at 20-25 °C for 3 h, and filtered. The filter cake was dried *in vacuo* to give the crystalline form VI (0.80 g) with 99% or more purity.

The crystalline form VI was characterized by XRPD, PLM, DSC and TGA. The locations and intensities of XRPD characteristic peaks are shown in Table 7, and the XRPD pattern is shown in FIG. 20. The PLM image (FIG. 21) shows that the sample is a crystal with irregular morphology and a particle size < 5 µm. The crystallinity is high. The DSC (FIG. 22) shows an endothermic peak with an initial temperature of 200 °C and an enthalpy value of 30 J/g after the solvent in the sample was removed, which indicates melting of the sample. The TGA (FIG. 22) of the sample shows a weight loss of 9.5% from room temperature to 130 °C, which corresponds to a broad endothermic peak in the DSC pattern and indicates removal of the water or solvent in the sample. After the water and solvent were removed at 40-50 °C, the crystalline form VI was transformed into the crystalline form VII (FIG. 20).

In the XRPD pattern of the crystalline form VI expressed in terms of 2θ angles, the 2θ values are shown in Table 7:

**Table 7. XRPD characteristic peaks of crystalline form VI**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 3.475 | 25.4043 | 0.9 | 20 | 18.375 | 4.8242 | 20.1 |
| 2 | 4.537 | 19.4587 | 3.4 | 21 | 18.785 | 4.72 | 0.8 |
| 3 | 5.232 | 16.8772 | 39.2 | 22 | 19.493 | 4.5501 | 0.7 |
| 4 | 5.627 | 15.6938 | 31.6 | 23 | 20.247 | 4.3823 | 0.5 |
| 5 | 6.898 | 12.8029 | 44.5 | 24 | 20.871 | 4.2528 | 14.2 |
| 6 | 8.081 | 10.9313 | 12.9 | 25 | 21.406 | 4.1476 | 0.8 |
| 7 | 9.013 | 9.8038 | 7.1 | 26 | 22.186 | 4.0035 | 1.5 |
| 8 | 10.354 | 8.5366 | 100 | 27 | 22.458 | 3.9556 | 2.4 |
| 9 | 11.325 | 7.8067 | 2.2 | 28 | 22.913 | 3.878 | 1.3 |
| 10 | 12.43 | 7.1149 | 2.2 | 29 | 23.233 | 3.8254 | 2 |
| 11 | 13.164 | 6.7202 | 4.3 | 30 | 23.826 | 3.7315 | 0.6 |
| 12 | 13.767 | 6.427 | 20.6 | 31 | 24.546 | 3.6237 | 1.3 |
| 13 | 14.926 | 5.9306 | 0.8 | 32 | 25.401 | 3.5036 | 10.1 |
| 14 | 15.775 | 5.6131 | 12.5 | 33 | 25.848 | 3.4441 | 20.9 |
| 15 | 16.262 | 5.4462 | 19.2 | 34 | 26.306 | 3.385 | 9.3 |
| 16 | 16.906 | 5.2401 | 0.3 | 35 | 28.947 | 3.082 | 1.1 |
| 17 | 17.273 | 5.1294 | 3 | 36 | 29.64 | 3.0114 | 1.2 |
| 18 | 17.746 | 4.994 | 2.2 | 37 | 31.611 | 2.828 | 1 |
| 19 | 18.139 | 4.8866 | 20.9 | | | | |

### Example 6

### Preparation of crystalline form VI:

Compound A (1 g) was added to butanone (10 mL). The mixture was stirred at 20-25 °C for 3 h, and filtered. The filter cake was dried *in vacuo* to give the crystalline form VI (0.85 g) with 99% or more purity.

The XRPD pattern of the crystalline form VI is shown in FIG. 20.

### Example 7

### Preparation of crystalline form VII:

Compound A (1.5 kg) was added to ethanol (7.5 L). The mixture was stirred at 70-75 °C for 1 h until it became clear, and cooled to 40-45 °C. Isopropyl acetate (15 L) was added in batches. The mixture was concentrated *in vacuo* to remove ethanol. Isopropyl acetate was added multiple times to facilitate the removal of ethanol, until the volume ratio of ethanol to isopropyl acetate was less than 5%. Isopropyl acetate was added to maintain the total volume of the system at 12 L. The mixture was cooled to 20-25 °C, stirred for 3 h, and filtered. The filter cake was dried *in vacuo* to give the crystalline form VII (1.4 kg) with 99% or more purity.

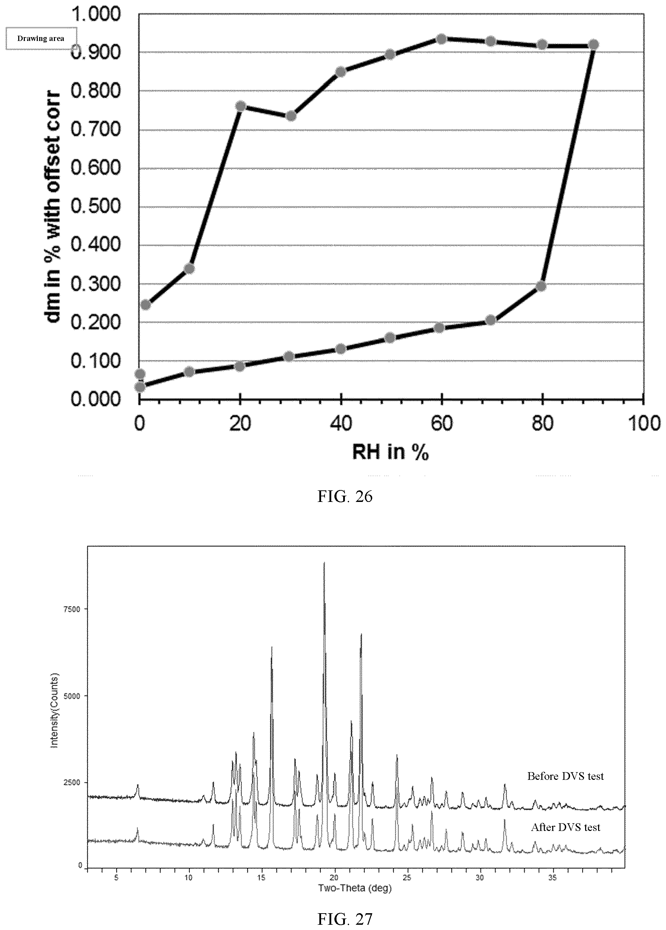

The crystalline form VII was characterized by XRPD, PLM, DSC, TGA and DVS. The locations and intensities of XRPD characteristic peaks are shown in Table 8, and the XRPD pattern is shown in FIG. 23. The PLM image (FIG. 24) shows that the sample is a crystal with irregular morphology and a particle size < 5 µm. The crystallinity is high. The DSC (FIG. 25) of the sample shows an endothermic peak with an initial temperature of 200 °C and an enthalpy value of 102 J/g, which indicates melting of the sample. The TGA (FIG. 25) of the sample shows almost no weight loss below 200 °C. The DVS results (FIG. 26) show slight hygroscopicity at only 0.29% within a range of 0-80% RH, and the crystalline form after the DVS test remained the same as that before the DVS test (FIG. 27).

In the XRPD pattern of the crystalline form VII expressed in terms of 2θ angles, the 2θ values are shown in Table 8:

**Table 8. XRPD characteristic peaks of crystalline form VII**

| Peak number | 2θ [ ° ] | d [ Å ] | Relative intensity (%) | Peak number | 2θ [ ° ] | d [ Å ] | Relative intensity (%) |
|---|---|---|---|---|---|---|---|
| 1 | 6.413 | 13.7711 | 8 | 25 | 25.82 | 3.4477 | 1.7 |
| 2 | 10.957 | 8.0679 | 1.1 | 26 | 26.135 | 3.4069 | 6.1 |
| 3 | 11.625 | 7.6058 | 3.1 | 27 | 26.385 | 3.3751 | 3 |
| 4 | 12.939 | 6.8362 | 25.9 | 28 | 26.621 | 3.3457 | 10.5 |
| 5 | 13.176 | 6.7139 | 12.7 | 29 | 27.328 | 3.2608 | 0.2 |
| 6 | 13.451 | 6.5772 | 5.9 | 30 | 27.618 | 3.2272 | 9.2 |
| 7 | 14.409 | 6.142 | 16.3 | 31 | 28.436 | 3.1362 | 2 |
| 8 | 14.567 | 6.0757 | 9.9 | 32 | 28.724 | 3.1054 | 4.6 |
| 9 | 15.644 | 5.6599 | 41.4 | 33 | 29.431 | 3.0323 | 1.4 |
| 10 | 17.246 | 5.1374 | 18.2 | 34 | 29.825 | 2.9932 | 5.9 |
| 11 | 17.522 | 5.0573 | 6.2 | 35 | 30.35 | 2.9426 | 4.9 |
| 12 | 17.655 | 5.0195 | 4.4 | 36 | 30.624 | 2.9169 | 0.8 |
| 13 | 18.783 | 4.7205 | 1.4 | 37 | 31.637 | 2.8258 | 11.1 |
| 14 | 19.255 | 4.6058 | 100 | 38 | 32.123 | 2.7841 | 2.6 |
| 15 | 19.732 | 4.4955 | 3.4 | 39 | 32.856 | 2.7236 | 1.5 |
| 16 | 19.94 | 4.4492 | 5.1 | 40 | 33.763 | 2.6526 | 3.9 |
| 17 | 21.106 | 4.2058 | 10.3 | 41 | 34.092 | 2.6277 | 1.4 |
| 18 | 21.75 | 4.0828 | 41 | 42 | 34.973 | 2.5635 | 4.4 |
| 19 | 22.001 | 4.0367 | 3.4 | 43 | 35.367 | 2.5359 | 2.8 |
| 20 | 22.539 | 3.9415 | 12.6 | 44 | 35.841 | 2.5033 | 2.9 |
| 21 | 24.231 | 3.67 | 18 | 45 | 37.7 | 2.3841 | 1.2 |
| 22 | 24.704 | 3.6008 | 0.7 | 46 | 38.242 | 2.3515 | 1.6 |
| 23 | 25.058 | 3.5508 | 3.6 | 47 | 39.185 | 2.2971 | 2.1 |
| 24 | 25.308 | 3.5162 | 7 | 48 | 39.307 | 2.2902 | 2.1 |

### Example 8

### Competitive slurrying experiment

A competitive slurrying experiment was carried out by slurrying a certain amount of each of the crystalline forms I, III, VII and IX with butanone and isopropyl acetate. The results are shown in Table 9, showing that the crystalline form VII can be produced from various slurrying processes.

**Table 9. Results of the competitive slurrying experiment of various crystalline forms**

| **Solvent** | **Mass (mg) Crystalline forms I + III + VII + IX** | **Volume (mL)** | **Temperature** | **XRPD results** |
|---|---|---|---|---|
| Butanone | 4.97 + 4.99 + 5.08 + 5.00 | 0.5 | 20°C | Crystalline form VII |
| Butanone | 4.97 + 5.05 + 4.98 + 4.96 | 0.4 | 50°C | Crystalline form VII |
| Isopropyl acetate | 4.98 + 5.06 + 4.96 + 5.05 | 1 | 20°C | Crystalline form VII |
| Isopropyl acetate | 5.07 + 4.98 + 4.93 + 4.95 | 1 | 50°C | Crystalline form VII |

### Stability studies of crystalline forms III and VII

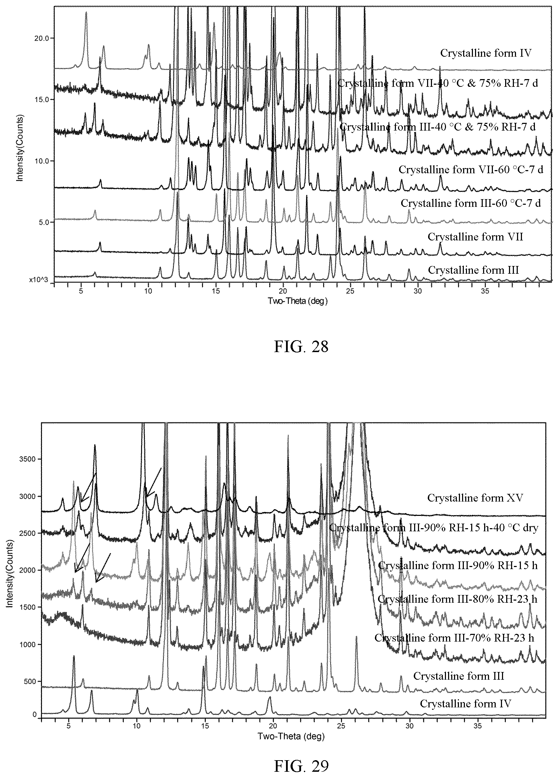

The crystalline forms III and VII were subjected to solid stability and chemical stability experiments under conditions of 60 °C/sealed and 40 °C/75% RH/open for 7 days. The results show that the crystalline forms III and VII were physically and chemically stable after being let stand at 60 °C (sealed) for 7 days, and were chemically stable after being let stand at 40 °C/75% RH (open) for 7 days. However, a small amount of hydrate crystalline form IV formed in both of them after they were let stand at 40 °C/75% RH (open) for 7 days. The amount of the crystalline form IV that formed in the crystalline form III is slightly greater than that in the crystalline form VII. The results of the experiment are shown in Table 10 and FIG. 28.

**Table 10. HPLC results for stability samples**

| **Sample** | **Initial purity (Area%)** | **Purity-7 days (Area%)** | | **XRPD** | |
|---|---|---|---|---|---|
| | | **40°C/75%RH** | **60°C** | **40°C/75%RH** | **60°C** |
| Crystalline form III | 99.96 | 99.96 | 99.97 | Crystalline form VII + crystalline form IV (small amount) | Remains unchanged |
| Crystalline form VII | 99.92 | 99.93 | 99.94 | Crystalline form VII + Crystalline form IV (tiny amount) | Remains unchanged |

### Test for effects of humidity on crystalline forms III and VII

It is known from the results of the stability experiment that a small amount of hydrate crystalline form IV formed in both of the crystalline forms III and VII after they were let stand at 40 °C/75% RH (open) for 7 days. Therefore, they were further studied at 40 °C for the effects of humidity.

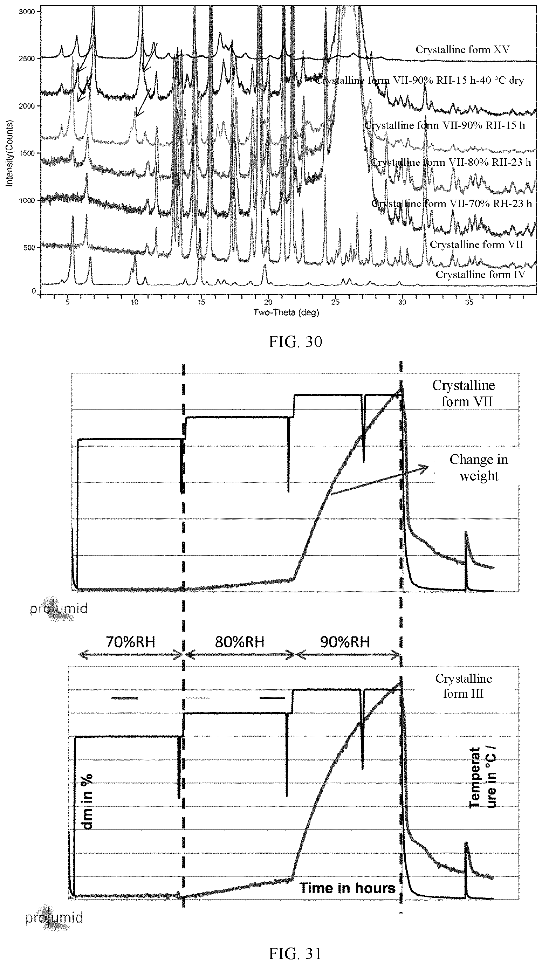

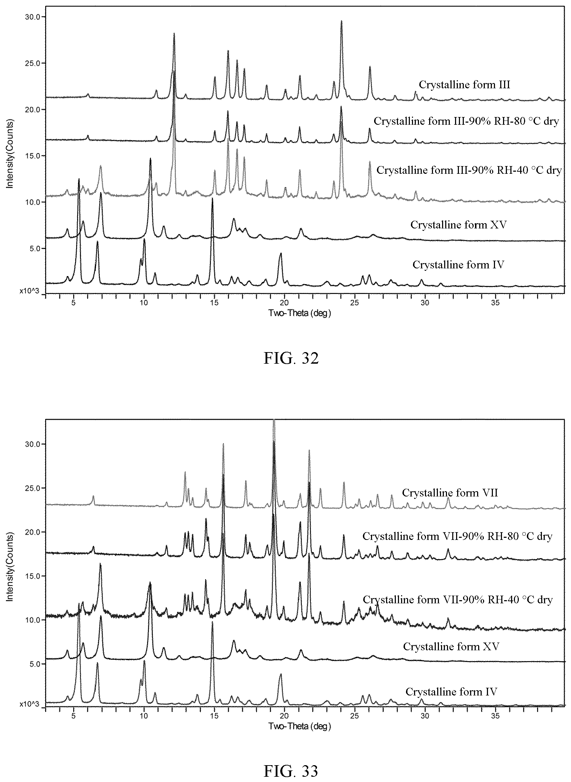

The results of the experiment are all shown in Table 11 and FIGs. 29-33. The crystalline forms III (FIG. 29) and VII (FIG. 30) were physically stable after being let stand at 70% RH for 23 h; a small amount of hydrate crystalline form IV formed in both of them after they were let stand at 80% and 90% RH for 23 h, and the hydrate crystalline form IV was transformed into a hydrate crystalline form XV after they were dried *in vacuo* at 40 °C overnight; however, the hydrate crystalline form IV can be completely removed by drying them *in vacuo* at 80 °C for 3 days (FIGs. 32 and 33).

According to the results of the humidity test, the crystalline forms III and VII are physically stable at 70% RH or less, while a small amount of hydrate crystalline form IV will form at 70% RH or more, and the hydrate can be removed by drying them *in vacuo* at 80 °C.

**Table 11. Results of the test for effects of humidity on crystalline forms III and VII**

| **Sample** | **XRPD** | | | |
|---|---|---|---|---|
| | **70%RH After 23 h** | **80%RH After 23 h** | **90%RH After 15 h** | **90%RH After 15 h-dry** |
| Crystalline form III | Remains unchanged | Crystalline form III + crystalline form IV (small amount) | Crystalline form III + crystalline form IV (small amount) | Crystalline form III + crystalline form XV (small amount) |
| Crystalline | Remains | Crystalline form | Crystalline form VII | Crystalline form |
| form VII | unchanged | VII + crystalline form IV (tiny amount) | + crystalline form IV (small amount) | VII + crystalline form XV (small amount) |

### Solubility test

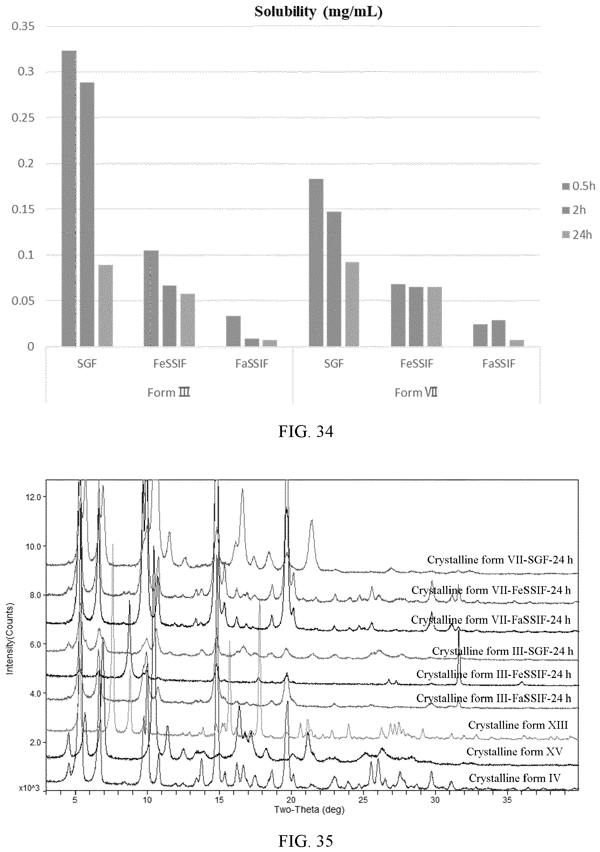

The solubility of both the crystalline forms III and VII increased with decreasing pH value of the biological medium. Both the crystalline forms were most soluble in SGF (0.323 mg/mL VS 0.183 mg/mL @ 0.5 h) and least soluble in FaSSIF (0.034 mg/mL VS 0.025 mg/mL @ 0.5 h). The solubility of the crystalline form III at 0.5 h is 1.5 times that of the crystalline form VII in all the three biological media. Both the crystalline forms degraded to a certain extent as the stirring continued in the solubility test. The results of the experiment are all shown in Table 12 and FIGs. 34-35. The crystalline forms of the crystalline forms III and VII in all the three biological media changed. The crystalline form III was transformed into the crystalline form IV in both FaSSIF and SGF, and into a crystal mixture of the crystalline forms IV and XIII in FeSSIF. The crystalline form VII was transformed into the crystalline form IV in both FaSSIF and FeSSIF, and into a crystal mixture of the crystalline forms IV and XV in SGF. The HPLC purity of the crystalline forms III and VII decreased to a certain extent in all the three biological media (calculated as 100% HPLC purity before being let stand in the biological media). Thus, the compound is unstable in biological media (Table 13).

**Table 12. Results of the solubility test**

| **Sample** | **Vehicle** | **Solubility (mg/mL)** | | | **pH** | | | **XRPD** |
|---|---|---|---|---|---|---|---|---|
| | | **0.5h** | **2h** | **24h** | **0.5h** | **2h** | **24h** | |
| Crystalline form III | SGF | 0.323 | 0.289 | 0.089 | 1.09 | 1.07 | 1.24 | Crystalline form IV |
| | FeSSIF | 0.105 | 0.067 | 0.058 | 4.88 | 4.92 | 4.94 | Crystalline forms IV + XIII |
| | FaSSIF | 0.034 | 0.009 | 0.007 | 6.47 | 6.47 | 6.45 | Crystalline form IV |
| Crystalline | SGF | 0.183 | 0.147 | 0.092 | 1.23 | 1.16 | 1.26 | Crystalline |
| form VII | | | | | | | | forms IV + XV |
| | FeSSIF | 0.068 | 0.065 | 0.065 | 4.86 | 4.90 | 4.91 | Crystalline form IV |
| | FaSSIF | 0.025 | 0.029 | 0.007 | 6.42 | 6.44 | 6.48 | Crystalline form IV |

**Table 13. HPLC results of the solubility test**

| **Sample** | **Vehicle** | **HPLC (24h)** |
|---|---|---|
| Crystalline form III | SGF | 99.69% |
| | FeSSIF | 96.89% |
| | FaSSIF | 97.99% |
| Crystalline form VII | SGF | 99.46% |
| | FeSSIF | 93.13% |
| | FaSSIF | 93.49% |

The examples of the present disclosure have been described above. However, the present disclosure is not limited to the above examples. Any modification, equivalent, improvement and the like made without departing from the spirit and principle of the present disclosure shall fall within the protection scope of the present disclosure.

## Claims

1. A polymorph of 2-((2-(*trans*-4-hydroxy-*cis*-4-methylcyclohexyl)-6-methoxy-2*H*-indazol-5-yl)carbamoyl)-6-m ethylpyridine 1-oxide shown as compound A of the following formula:

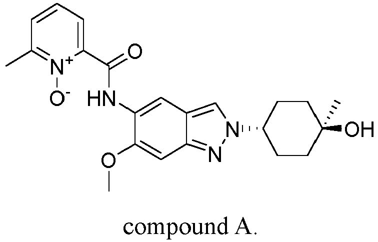

2. The polymorph according to claim 1, wherein the polymorph is a crystalline form III **characterized by** X-ray powder diffraction peaks measured using Cu-Kα radiation at 2θ angles of 12.15±0.20°, 15.98±0.20°, 16.62±0.20°, 17.14±0.20°, 24.32±0.20° and 26.08±0.20°;
preferably, the crystalline form III is an anhydrate of compound A;
preferably, the crystalline form III has an X-ray powder diffraction pattern substantially as shown in FIG. 4;
preferably, differential scanning calorimetry (DSC) analysis of the crystalline form III shows a first endothermic peak at a peak temperature raised to near 188.81 °C;
preferably, thermogravimetric analysis (TGA) of the crystalline form III shows almost no weight loss below 180 °C;
preferably, the crystalline form III has a DSC-TGA pattern substantially as shown in FIG. 8;
preferably, the crystalline form III is a crystal with irregular morphology; preferably, the crystalline form III has a particle size of less than 5 µm; preferably, the crystalline form III has a PLM image substantially as shown in FIG. 6;
preferably, the crystalline form III has a purity of 95% or more.

3. The polymorph according to claim 1, wherein the polymorph is a crystalline form VII **characterized by** X-ray powder diffraction peaks measured using Cu-Kα radiation at 2θ angles of 12.94±0.20°, 14.41±0.20°, 15.64±0.20°, 17.25±0.20°, 21.75±0.20° and 24.23±0.20°;
preferably, the crystalline form VII is an anhydrate of compound A;
preferably, the crystalline form VII has an X-ray powder diffraction pattern substantially as shown in FIG. 23;
preferably, differential scanning calorimetry (DSC) analysis of the crystalline form VII shows an endothermic peak at a peak temperature raised to near 201.07 °C;
preferably, thermogravimetric analysis (TGA) of the crystalline form VII shows almost no weight loss below 200 °C, preferably, almost no weight loss below 180 °C;
preferably, the crystalline form VII has a DSC-TGA pattern substantially as shown in FIG. 25;
preferably, the crystalline form VII is a crystal with irregular morphology; preferably, the crystalline form VII has a particle size of less than 5 µm; preferably, the crystalline form VII has a PLM image substantially as shown in FIG. 24;
preferably, the crystalline form VII has a purity of 95% or more.

4. The polymorph according to claim 1, wherein the polymorph is a crystalline form I **characterized by** X-ray powder diffraction peaks measured using Cu-Kα radiation at 2θ angles of 11.85±0.20°, 15.86±0.20°, 16.57±0.20°, 17.68±0.20°, 20.99±0.20° and 23.99±0.20°;
preferably, the crystalline form I has an X-ray powder diffraction pattern substantially as shown in FIG. 1.

5. The polymorph according to claim 1, wherein the polymorph is a crystalline form II **characterized by** X-ray powder diffraction peaks measured using Cu-Kα radiation at 2θ angles of 13.49±0.20°, 17.51±0.20°, 17.72±0.20°, 20.97±0.20°, 23.67±0.20° and 27.32±0.20°;
preferably, the crystalline form II has an X-ray powder diffraction pattern substantially as shown in FIG. 4.

6. The polymorph according to claim 1, wherein the polymorph is a crystalline form IV **characterized by** X-ray powder diffraction peaks measured using Cu-Kα radiation at 2θ angles of 5.38±0.20°, 6.68±0.20°, 9.76±0.20°, 19.69±0.20°, 27.48±0.20° and 29.65±0.20°;
preferably, the crystalline form IV has an X-ray powder diffraction pattern substantially as shown in FIG. 11.

7. The polymorph according to claim 1, wherein the polymorph is a crystalline form V **characterized by** X-ray powder diffraction peaks measured using Cu-Kα radiation at 2θ angles of 7.11±0.20°, 9.62±0.20°, 14.07±0.20°, 19.23±0.20°, 21.59±0.20° and 25.65±0.20°;
preferably, the crystalline form V has an X-ray powder diffraction pattern substantially as shown in FIG. 14.

8. The polymorph according to claim 1, wherein the polymorph is a crystalline form IX **characterized by** X-ray powder diffraction peaks measured using Cu-Kα radiation at 2θ angles of 8.26±0.20°, 9.33±0.20°, 11.07±0.20°, 16.81±0.20°, 20.73±0.20° and 21.01±0.20°;
preferably, the crystalline form IX has an X-ray powder diffraction pattern substantially as shown in FIG. 17.

9. The polymorph according to claim 1, wherein the polymorph is a crystalline form VI **characterized by** X-ray powder diffraction peaks measured using Cu-Kα radiation at 2θ angles of 5.23±0.20°, 5.63±0.20°, 6.90±0.20°, 13.77±0.20°, 18.14±0.20° and 25.85±0.20°;
preferably, the crystalline form VI has an X-ray powder diffraction pattern substantially as shown in FIG. 20.

10. A preparation method for the crystalline form III according to claim 2 comprising the following step: heating a crystalline form II to give the crystalline form III, wherein
the crystalline form II is **characterized by** X-ray powder diffraction peaks measured using Cu-Kα radiation at 2θ angles of 13.49±0.20°, 17.51±0.20°, 17.72±0.20°, 20.97±0.20°, 23.67±0.20° and 27.32±0.20°;
preferably, the crystalline form II is a toluene solvate of compound A.

11. A preparation method for the crystalline form VII according to claim 3 selected from any one of the following methods:
method 1, comprising the following steps:
mixing compound A with a first organic solvent, stirring the mixture at room temperature until complete dissolution is achieved, and performing filtration and drying to give the crystalline form VII, wherein
the first organic solvent is selected from one, two or more of butanone, isopropyl acetate, ethanol and n-butanol;
method 2, comprising the following step: heating a crystalline form VI to give the crystalline form VII, wherein
the crystalline form VI is **characterized by** X-ray powder diffraction peaks measured using Cu-Kα radiation at 2θ angles of 5.23±0.20°, 5.63±0.20°, 6.90±0.20°, 13.77±0.20°, 18.14±0.20° and 25.85±0.20°;
preferably, the crystalline form VI is a methanol solvate/hydrate of compound A;
method 3, comprising the following steps:
mixing compound A with an alcohol solvent, heating and stirring the system until complete dissolution is achieved, and cooling the system; subsequently, adding an organic acid ester to the system, and concentrating the system *in vacuo* until a volume ratio of the alcohol solvent to the organic acid ester in the system is less than 5%; and supplementing the system with the organic acid ester, cooling again and then stirring the system, and performing filtration and drying to give the crystalline form VII; wherein
preferably, the alcohol solvent is selected from ethanol and/or *n*-butanol;
preferably, the organic acid ester is selected from isopropyl acetate and/or ethyl acetate;
preferably, the system is heated to a temperature of 65-80 °C; and
method 4, comprising the following step: mixing and slurrying a mixture of a crystalline form I, a crystalline form III, the crystalline form VII and a crystalline form IX with a second organic solvent to give the crystalline form VII, wherein
the crystalline form I is **characterized by** X-ray powder diffraction peaks measured using Cu-Kα radiation at 2θ angles of 11.85±0.20°, 15.86±0.20°, 16.57±0.20°, 17.68±0.20°, 20.99±0.20° and 23.99±0.20°;
the crystalline form IX is **characterized by** X-ray powder diffraction peaks measured using Cu-Kα radiation at 2θ angles of 8.26±0.20°, 9.33±0.20°, 11.07±0.20°, 16.81±0.20°, 20.73±0.20° and 21.01±0.20°;
preferably, a mass ratio of the crystalline form I to the crystalline form III to the crystalline form VII to the crystalline form IX is (0.9-1.1):(0.9-1.1):1:(0.9-1.1);
preferably, the second organic solvent is selected from one, two or more of butanone, ethyl acetate, isopropyl acetate, ethanol and *n*-butanol;
preferably, the mixture is slurried at a temperature of 15-60 °C.

12. A method for preserving the crystalline form III according to claim 2 or the crystalline form VII according to claim 3, wherein the crystalline form III or VII is placed at a relative humidity level of less than 75% RH;
preferably, the crystalline form III or VII is placed at a temperature of room temperature to 60 °C.

13. A pharmaceutical composition comprising the polymorph according to any one of claims 1-9.

14. A formulation comprising the polymorph according to any one of claims 1-9, and optionally a pharmaceutically acceptable excipient.

15. Use of the polymorph according to any one of claims 1-9 or the pharmaceutical composition according to claim 7 in preparing a medicament for preventing and/or treating an IRAK-mediated disease or condition, wherein
preferably, the IRAK-mediated disease or condition is selected from tumors, gout, systemic lupus erythematosus, multiple sclerosis, metabolic syndrome, atherosclerosis, myocardial infarction, sepsis, inflammatory bowel disease, asthma and allergy.

16. Use of the polymorph according to any one of claims 1-9 or the pharmaceutical composition according to claim 13 in preparing a medicament for preventing and/or treating a disease or condition associated with interleukin-1 receptor associated kinases.
